(19) Europäisches Patentamt
European Patent Office
Office européen des brevets

(11) Veröffentlichungsnummer : **0 481 354 B1**

(12) **EUROPÄISCHE PATENTSCHRIFT**

(45) Veröffentlichungstag der Patentschrift : **03.08.94 Patentblatt 94/31**

(21) Anmeldenummer : **91117262.5**

(22) Anmeldetag : **10.10.91**

(51) Int. Cl.[5] : **C07C 251/52,** A01N 43/16, A01N 43/18, C07C 251/42, C07C 323/47, C07C 239/20, C07D 309/06, C07D 209/48, C07D 335/02

(54) **Ungesättigte Cyclohexenonoximether.**

(30) Priorität : **19.10.90 DE 4033193**
**27.11.90 DE 4037636**

(43) Veröffentlichungstag der Anmeldung : **22.04.92 Patentblatt 92/17**

(45) Bekanntmachung des Hinweises auf die Patenterteilung : **03.08.94 Patentblatt 94/31**

(84) Benannte Vertragsstaaten : **AT BE CH DE ES FR GB IT LI NL**

(56) Entgegenhaltungen :
**EP-A- 0 230 235**
**EP-A- 0 254 514**
**EP-A- 0 341 048**
**EP-A- 0 368 227**

(73) Patentinhaber : **BASF Aktiengesellschaft**
**Carl-Bosch-Strasse 38**
**D-67063 Ludwigshafen (DE)**

(72) Erfinder : **Kast, Juergen, Dr.**
**Kastanienstrasse 24**
**W-6737 Boehl-Iggelheim (DE)**
Erfinder : **Meyer, Norbert, Dr.**
**Dossenheimer Weg 22**
**W-6802 Ladenburg (DE)**
Erfinder : **Misslitz, Ulf, Dr.**
**Am Haerzl 40**
**W-6730 Neustadt (DE)**
Erfinder : **Harreus, Albrecht, Dr.**
**Teichgasse 13**
**W-6700 Ludwigshafen (DE)**
Erfinder : **Rang, Harald, Dr.**
**Maximilianstrasse 30**
**W-6700 Ludwigshafen (DE)**
Erfinder : **Gerber, Matthias, Dr.**
**Ritterstrasse 3**
**W-6704 Mutterstadt (DE)**
Erfinder : **Walter, Helmut, Dr.**
**Gruenstadter Strasse 82**
**W-6719 Obrigheim (DE)**
Erfinder : **Westphalen, Karl-Otto, Dr.**
**Mausbergweg 58**
**W-6720 Speyer (DE)**

**EP 0 481 354 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue ungesättigte Cyclohexenonoximether der allgemeinen Formel I

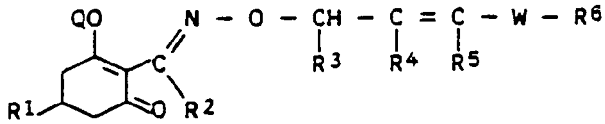

I

wobei die Variablen die folgende Bedeutung haben:

Q

Wasserstoff, eine $C_1$-$C_6$-Alkylcarbonylgruppe, die Benzoylgruppe, ein Alkalimetall- oder Erdalkalimetallion, ein Ammoniumion, dessen Stickstoffatom ein bis vier $C_1$-$C_4$-Alkyl-, Phenyl- und/oder Benzylsubstituenten tragen kann, ein Phosphonium-, Sulfonium- oder Sulfoxoniumion oder ein Äquivalent eines Übergangsmetallkations;

W

eine Gruppe -C≡C- oder -CH=CH-;

$R^1$

eine $C_1$-$C_4$-Alkoxy-$C_1$-$C_6$-alkyl- oder $C_1$-$C_4$-Alkythio-$C_1$-$C_6$-alkylgruppe, eine $C_3$-$C_7$-Cycloalkyl- oder $C_5$-$C_7$-Cycloalkenylgruppe, eine 6-gliedrige heterocyclische Gruppe mit einem oder zwei nicht benachbarten Sauerstoff- und/oder Schwefelatomen, die gesättigt oder partiell ungesättigt sein kann, wobei die cyclischen Gruppen noch einen bis drei der folgenden Reste tragen können: Hydroxyl, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy oder $C_1$-$C_4$-Alkylthio;

ein 5-gliedriger gesättigter Heterocyclus mit einem oder zwei Sauerstoffund/oder Schwefelatomen als Heteroatome, der noch einen bis drei der folgenden Reste tragen kann: $C_1$-$C_4$-Alkyl, partiell oder vollständig halogeniertes $C_1$-$C_4$-Alkyl, $C_1$-$C_4$-Alkoxy oder $C_1$-$C_4$-Alkylthio;

eine 5-gliedrige heteroaromatische Gruppe mit einem oder zwei Stickstoffatomen und/oder einem Sauerstoff- oder Schwefelatom, die noch einen bis drei der folgenden Reste tragen kann: Cyano, Halogen, $C_1$-$C_4$-Alkyl, partiell oder vollständig halogeniertes $C_1$-$C_4$-Alkyl, $C_2$-$C_6$-Alkenyl, partiell oder vollständig halogeniertes $C_2$-$C_6$-Alkenyl, $C_1$-$C_4$-Alkoxy, $C_1$-$C_4$-Alkylthio, $C_2$-$C_6$-Alkenyloxy oder $C_1$-$C_4$-Alkoxy-$C_1$-$C_4$-alkyl;

die Phenyl- oder Pyridylgruppe oder eine durch einen Rest $R^7$-X- substituierte $C_1$-$C_6$-Alkylgruppe, wobei X Sauerstoff, Schwefel, -SO- oder -$SO_2$- und $R^7$ $C_1$-$C_4$-Alkyl, Phenyl oder 5- oder 6-gliedriges Heteroaryl mit einem bis drei Heteroatomen, ausgewählt aus einer Gruppe von einem Sauerstoff- oder Schwefelatom und drei Stickstoffatomen, ausgenommen Verbindungen mit drei gleichzeitig benachbarten Heteroatomen im Heterocyclus, bedeutet und wobei der Aromat oder Heteroaromat dieser Gruppen noch einen bis drei der folgenden Substituenten tragen kann: Nitro, Cyano, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, $C_1$-$C_4$-Alkoxy-$C_1$-$C_4$-alkyl, $C_1$-$C_4$-Alkylthio-$C_1$-$C_4$-alkyl, $C_3$-$C_6$-Alkenyloxy, $C_3$-$C_6$-Alkinyloxy oder -$NR^8R^9$, wobei $R^8$ Wasserstoff, $C_1$-$C_4$-Alkyl, $C_3$-$C_4$-Alkenyl oder $C_3$-$C_4$-Alkinyl und $R^9$ Wasserstoff, $C_1$-$C_4$-Alkyl, $C_3$-$C_4$-Alkenyl, $C_3$-$C_4$-Alkinyl, $C_1$-$C_6$-Alkylcarbonyl oder Benzoyl, das zusätzlich noch einen bis drei der folgenden Reste tragen kann: Nitro, Cyano, Halogen, $C_1$-$C_4$-Alkyl, partiell oder vollständig halogeniertes $C_1$-$C_4$-Alkyl, $C_1$-$C_4$-Alkoxy oder $C_1$-$C_4$-Alkylthio, bedeuten;

$R^2$

eine $C_1$-$C_6$-Alkylgruppe;

$R^3$

Wasserstoff oder eine $C_1$-$C_6$-Alkylgruppe;

$R^4$

Wasserstoff, Halogen oder eine $C_1$-$C_6$-Alkylgruppe;

$R^5$

Wasserstoff oder eine $C_1$-$C_6$-Alkylgruppe;

oder $R^3$ und $R^4$, $R^3$ und $R^5$ oder $R^4$ und $R^5$ bilden zusammen eine $C_2$-$C_4$-Alkylen- oder $C_2$-$C_4$-Alkenylenkette;

$R^6$

Wasserstoff, eine $C_1$-$C_6$-Alkylgruppe, eine partiell oder vollständig halogenierte $C_1$-$C_4$-Alkylgruppe, die noch einen Phenylrest tragen kann, eine $C_2$-$C_6$-Alkenylgruppe, eine $C_1$-$C_4$-Alkoxy-$C_1$-$C_4$-alkylgruppe, eine $C_1$-$C_4$-Alkylthio-$C_1$-$C_4$-alkylgruppe, eine $C_3$-$C_7$-Cycloalkyl- oder $C_5$-$C_7$-Cycloalkenylgruppe, die beide noch einen bis drei der folgenden Reste tragen können: Hydroxyl, $C_1$-$C_4$-Alkyl, partiell oder vollständig halogeniertes $C_1$-$C_4$-

Alkyl, $C_1$-$C_4$-Alkoxy, $C_1$-$C_4$-Alkoxy-$C_1$-$C_4$-alkyl oder $C_1$-$C_4$-Alkylthio-$C_1$-$C_4$-alkyl;
die Phenyl- oder die Pydridylgruppe, die beide noch einen bis drei der folgenden Reste tragen können: Nitro, Cyano, Hydroxyl, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, $C_1$-$C_4$-Alkoxy-$C_1$-$C_4$-alkyl oder $C_1$-$C_4$-Alkylthio-$C_1$-$C_4$-alkyl.

Außerdem betrifft die Erfindung herbizide Mittel, welche diese Verbindungen als wirksame Substanzen enthalten.

Des weiteren betrifft die Erfindung Hydroxylamine der Formel IIIb

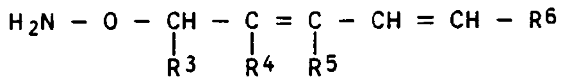

IIIb

und Phthalimide der allgemeinen Formel IX

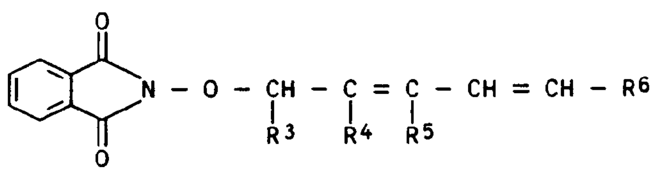

IX

als Zwischenprodukte.

Herbizide Cyclohexenonoximether vom Typ der Verbindungen I, die sich durch einen anderen Oximether-Teil und/oder einem anderen Substituenten in 5-Position des Cyclohexan-1,3-dion-Grundkörpers von den Verbindungen I unterscheiden, sind aus folgenden Druckschriften bekannt:

a) US-A 4 440 566 (Halogenalkenyl- und Benzylether);

b) US-A 4 249 937 (Alkyl- und Alkenylether);

c) EP-A 080 301, EP-A 238 021 und EP-A 125 094 (Benzylether und But-2-enylether);

d) EP-A 218 233 (But-2-enylether);

e) DE-A 38 38 309 (4-Phenylbutyl-, 4-Phenylbut-2-enyl- und 4-Phenylbut-3-enylether);

f) EP-A 253 537, EP-A 323 915, EP-A 254 514, JP-A 1006 255, JP-A 1013 068, JP-A 1013 064, JP-A 1013 066, JP-A 1016 759, JP-A 1029 355, JP-A 1031 756, JP-A 1153 637, JP-A 1157 947, JP-A 1157 948, JP-A 1157 949, JP-A 1157 950, JP-A 1168 664, JP-A 1180 869, JP-A 1180 870 und JP-A 1180 871 (Benzylether und Butenylether).

Es sind jedoch Verbindungen wünschenswert, die bei geringeren Aufwandmengen eine gute herbizide Wirkung gegen unerwünschte grasartige Pflanzen zeigen, ohne die Kulturpflanzen nennenswert zu schädigen.

Der Erfindung lagen daher neue Substanzen mit verbesserter herbizider Wirkung als Aufgabe zugrunde.

Demgemäß wurden die eingangs definierten ungesättigten Cyclohexenonoximether I gefunden.

Außerdem wurden herbizide Mittel gefunden, die diese Substanzen enthalten.

Im einzelnen haben die Variablen in den erfindungsgemäßen Verbindungen I die folgende Bedeutung:

Q

- Wasserstoff;
- eine verzweigte oder unverzweigte $C_1$-$C_6$-Alkylcarbonylgruppe wie Methylcarbonyl, Ethylcarbonyl, Propylcarbonyl, 1-Methylethyl-carbonyl, Butylcarbonyl, 1-Methylpropylcarbonyl, 2-Methylpropylcarbonyl, 1,1-Dimethylethylcarbonyl, Pentylcarbonyl, 1-Methylbutylcarbonyl, 2-Methylbutylcarbonyl, 3-Methylbutylcarbonyl, 1,1-Dimethylpropylcarbonyl, 1,2-Dimethylpropylcarbonyl, 2,2-Dimethylpropylcarbonyl, 1-Ethylpropylcarbonyl, Hexylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, 2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl und 1-Ethyl-2-methylpropylcarbonyl;
- die Benzoylgruppe;
- ein Alkalimetall- oder Erdalkalimetallion wie Natrium, Kalium, Calcium, Magnesium und Barium;
- ein Ammoniumion, dessen Stickstoffatom ein bis vier Substituenten tragen kann, ausgewählt aus einer Gruppe von vier $C_1$-$C_4$-Alkylsubstituenten wie Methyl, Ethyl, n-Propyl, Isopropyl, n-Butyl und tert.- Butyl, zwei Phenyl- und zwei Benzylsubstituenten, insbesondere ein Diisopropylammonium-, Tetramethylammonium-, Tetrabutylammonium- und Trimethylbenzylammoniumion;

- ein Phosphoniumion;
- ein Sulfoniumion, insbesondere ein Trialkylsulfoniumion;
- ein Sulfoxoniumion;
- ein Äquivalent eines Übergangsmetallkations, insbesondere Mangan, Eisen, Kupfer und Zink;

W

- eine Gruppe -C≡C- oder -CH=CH-;

$R^1$

- eine $C_3$-$C_7$-Cycloalkylgruppe wie Cyclopropyl, Cyclobutyl, Cyclopentyl und Cyclohexyl, eine $C_5$-$C_7$-Cycloalkenylgruppe wie Cyclopent-1-enyl, Cyclopent-2-enyl, Cyclopent-3-enyl, Cyclohex-1-enyl, Cyclohex-2-enyl, Cyclohex-3-enyl, Cyclohept-1-enyl, Cyclohept-2-enyl, Cyclohept-3-enyl, Cyclohept-4-enyl, oder eine 6-gliedrige heterocyclische Gruppe mit einem oder zwei nicht benachbarten Sauerstoff- und/oder Schwefelatomen, die gesättigt oder partiell ungesättigt sein kann, wie 5,6-Di-hydropyran-3-yl, 5,6-Dihydrothiopyran-3-yl, Tetrahydropyran-3-yl, Tetrahydropyran-4-yl, Tetrahydrothiopyran-3-yl und 1,3-Dioxepan-5-yl, wobei die cyclischen Gruppen noch einen bis drei der folgenden Reste tragen können: Hydroxyl, Halogen wie Fluor, Chlor, Brom und Jod, insbesondere Chlor und Brom, $C_1$-$C_4$-Alkyl wie Methyl, Ethyl, n-Propyl, Isopropyl, n-Butyl und tert.-Butyl, insbesondere Methyl und Isopropyl, partiell oder vollständig halogeniertes $C_1$-$C_4$-Alkyl wie Fluormethyl, Chlormethyl, Brommethyl, Difluormethyl, Trifluormethyl, Trichlormethyl, 1-Chlorethyl, Pentafluorethyl, 4-Chlorbut-1-yl und 2-Chlor1,1,2-trifluorethyl, insbesondere Difluormethyl und Trifluormethyl, $C_1$-$C_4$-Alkoxy wie Methoxy, Ethoxy, n-Propoxy, Isopropoxy, n-Butoxy und tert.-Butoxy, partiell oder vollständig halogeniertes $C_1$-$C_4$-Alkoxy wie Difluormethoxy, Trifluormethoxy und 1,1,2,2-Tetrafluorethoxy, insbesondere Difluormethoxy und Trifluormethoxy, oder $C_1$-$C_4$-Alkylthio wie Methylthio, Ethylthio, n-Propylthio, Isopropylthio, 1-Methylpropylthio, 2-Methylpropylthio, n-Butylthio und tert.-Butylthio, insbesondere Methylthio; besonders bevorzugt sind 1-Methylthio-cyclopropyl, Cyclohexyl, 4-Methylcyclohexyl, 3,4-Dihydroxycyclohexyl, Cyclohexenyl, 5,6-Dihydropyran-3-yl, Tetrahydropyran-3-yl, Tetrahydropyran-4-yl, Tetrahydrothiopyran-3-yl, 3,4-Dibromtetrahydropyran-3-yl, 2-Isopropyl-1,3-dioxepan-5-yl;
- ein 5-gliedriger gesättigter Heterocyclus mit einem oder zwei Sauerstoff- und/oder Schwefelatomen als Heteroatome, z.B. Tetrahydrofuran 1,3-Dioxolan-2-yl und 1,3-Dithiolan-2-yl, der noch einen bis drei der folgenden Reste tragen kann: $C_1$-$C_4$-Alkyl wie vorstehend genannt, insbesondere Methyl, partiell oder vollständig halogeniertes $C_1$-$C_4$-Alkyl wie vorstehend genannt, insbesondere Trifluormethyl, $C_1$-$C_4$-Alkoxy wie vorstehend genannt, insbesondere Methoxy und Ethoxy, oder $C_1$-$C_4$-Alkylthio wie vorstehend genannt, insbesondere Methylthio;
- eine 5-gliedrige heteroaromatische Gruppe mit einem oder zwei Stickstoffatomen und/oder einem Sauerstoff- oder Schwefelatom, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4- Oxadiazol-3-yl, 1,2,4-Oxadiazol-5-yl, 1,2,4- Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl, die noch einen bis drei der folgenden Reste tragen kann: Cyano, Halogen wie vorstehend genannt, insbesondere Fluor und Chlor, $C_1$-$C_4$-Alkyl wie vorstehend genannt, partiell oder vollständig halogeniertes $C_1$-$C_4$-Alkyl wie vorstehend genannt, insbesondere Trifluormethyl, $C_2$-$C_6$-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;

insbesondere Ethenyl, Allyl und 1-Methylethenyl, partiell oder vollständig halogeniertes $C_2$-$C_6$-Alkenyl

wie 3-Chlorpropenyl und 2,3,3-Trichlorpropenyl;

$C_1$-$C_4$-Alkoxy wie vorstehend genannt, insbesondere Methoxy und Ethoxy, $C_1$-$C_4$-Alkylthio wie vorstehend genannt, insbesondere Methylthio, $C_2$-$C_6$-Alkenyloxy wie Ethenyloxy, 1-Propenyloxy, 2-Propenyloxy, 1-Methylethenyloxy, 1-Butenyloxy, 2-Butenyloxy, 3-Butenyloxy, 1-Methyl-1-propenyloxy, 2-Methyl-1-propenyloxy, 1-Methyl-2-propenyloxy, 2-Methyl-2-propenyloxy, 1-Pentenyloxy, 2-Pentenyloxy, 3-Pentenyloxy, 4-Pentenyloxy, 1-Methyl-1-butenyloxy, 2-Methyl-1-butenyloxy, 3-Methyl-1-butenyloxy, 1-Methyl-2-butenyloxy, 2-Methyl-2-butenyloxy, 3-Methyl-2-butenyloxy, 1-Methyl-3-butenyloxy, 2-Methyl-3-butenyloxy, 3-Methyl-3-butenyloxy, 1,1-Dimethyl-2-propenyloxy, 1,2-Dimethyl-1-propenyloxy, 1,2-Dimethyl-2-propenyloxy, 1-Ethyl-1-propenyloxy, 1-Ethyl-2-propenyloxy, 1-Hexenyloxy, 2-Hexenyloxy, 3-Hexenyloxy, 4-Hexenyloxy, 5-Hexenyloxy, 1-Methyl-1-pentenyloxy, 2-Methyl-1-pentenyloxy, 3-Methyl-1-pentenyloxy, 4-Methyl-1-pentenyloxy, 1-Methyl-2-pentenyloxy, 2-Methyl-2-pentenyloxy, 3-Methyl-2-pentenyloxy, 4-Methyl-2-pentenyloxy, 1-Methyl-3-pentenyloxy, 2-Methyl-3-pentenyloxy, 3-Methyl-3-pentenyloxy, 4-Methyl-3-pentenyloxy, 1-Methyl-4-pentenyloxy, 2-Methyl-4-pentenyloxy, 3-Methyl-4-pentenyloxy, 4-Methyl-4-pentenyloxy, 1, 1-Dimethyl-2-butenyloxy, 1,1-Di-methyl-3-butenyloxy, 1,2-Dimethyl-1-butenyloxy, 1,2-Dimethyl-2-butenyloxy, 1,2-Dimethyl-3-butenyloxy, 1,3-Dimethyl-1-butenyloxy, 1,3-Dimethyl-2-butenyloxy, 1,3-Dimethyl-3-butenyloxy, 2,2-Dimethyl-3-butenyloxy, 2,3-Dimethyl-1-butenyloxy, 2,3-Dimethyl-2-butenyloxy, 2,3-Dimethyl-3-butenyloxy, 3,3-Dimethyl-1-butenyloxy, 3,3-Dimethyl-2-butenyloxy, 1-Ethyl-1-butenyloxy, 1-Ethyl-2-butenyloxy, 1-Ethyl-3-butenyloxy, 2-Ethyl-1-butenyloxy, 2-Ethyl-2-butenyloxy, 2-Ethyl-3-butenyloxy, 1,1,2-Trimethyl-2-propenyloxy, 1-Ethyl-1-methyl-2-propenyloxy, 1-Ethyl-2-methyl-1-propenyloxy und 1-Ethyl-2-methyl-2-propenyloxy, vorzugsweise Prop-2-enyloxy, oder $C_1$-$C_4$-Alkoxy-$C_1$-$C_4$-alkyl wie Methoxymethyl, Methoxyethyl, Ethoxymethyl, Ethoxyethyl, insbesondere Methoxymethyl und Ethoxymethyl;

- die Phenyl- oder Pyridylgruppe oder eine durch einen Rest $R^7$-X- substituierte $C_1$-$C_6$-Alkylgruppe, wobei X Sauerstoff, Schwefel, -SO- oder -$SO_2$- und $R^7$ $C_1$-$C_4$-Alkyl, Phenyl oder 5- oder 6-gliedriges Heteroaryl mit einem bis drei Heteroatomen, ausgewählt aus einer Gruppe von einem Sauerstoff- oder Schwefelatom und drei Stickstoffatomen, ausgenommen Verbindungen mit drei gleichzeitig benachbarten Heteroatomen im Heterocyclus, bedeutet und wobei der Aromat oder Heteroaromat dieser Gruppen noch einen bis drei der folgenden Substituenten tragen kann: Nitro, Cyano, Halogen wie vorstehend genannt, insbesondere Fluor und Chlor, $C_1$-$C_4$-Alkyl wie vorstehend genannt, partiell oder vollständig halogeniertes $C_1$-$C_4$-Alkyl wie vorstehend genannt, insbesondere Difluormethyl und Trifluormethyl, $C_1$-$C_4$-Alkoxy wie vorstehend genannt, insbesondere Methoxy und Ethoxy, partiell oder vollständig halogeniertes $C_1$-$C_4$-Alkoxy wie vorstehend genannt, insbesondere Difluormethoxy und Trifluormethoxy, $C_1$-$C_4$-Alkylthio wie vorstehend genannt, insbesondere Methylthio, $C_1$-$C_4$-Alkoxy-$C_1$-$C_4$-alkyl wie vorstehend genannt, insbesondere Methoxymethyl und Ethoxymethyl, $C_1$-$C_4$-Alkylthio-$C_1$-$C_4$-alkyl wie vorstehend genannt, insbesondere Methylthiomethyl, $C_3$-$C_6$-Alkenyloxy wie Prop-2-enyloxy und But-2-enyloxy, $C_3$-$C_6$-Alkinyloxy wie Prop-2-inyloxy und But-2-inyloxy, oder -$NR^8R^9$, wobei $R^8$ Wasserstoff, $C_1$-$C_4$-Alkyl wie vorstehend genannt, $C_3$-$C_4$-Alkenyl wie Prop-2-enyl und But-2-enyl oder $C_3$-$C_4$-Alkinyl wie Prop-2-inyl und But-2-inyl und $R^9$ Wasserstoff, $C_1$-$C_4$-Alkyl wie vorstehend genannt, $C_3$-$C_4$-Alkenyl wie vorstehend genannt, $C_3$-$C_4$-Alkinyl wie vorstehend genannt, $C_1$-$C_6$-Alkylcarbonyl wie vorstehend genannt, oder Benzoyl, das zusätzlich noch einen bis drei der folgenden Reste tragen kann: Nitro, Cyano, Halogen wie vorstehend genannt, insbesondere Fluor und Chlor, $C_1$-$C_4$-Alkyl wie vorstehend genannt, partiell oder vollständig halogeniertes $C_1$-$C_4$-Alkyl wie vorstehend genannt, insbesondere Trifluormethyl, $C_1$-$C_4$-Alkoxy wie vorstehend genannt, insbesondere Methoxy und Ethoxy oder $C_1$-$C_4$-Alkylthio wie vorstehend genannt, insbesondere Methylthio, bedeuten;

$R^2$

- eine verzweigte oder unverzweigte $C_1$-$C_6$-Alkylgruppe, insbesondere eine $C_1$-$C_4$-Alkylgruppe wie vorstehend genannt;

$R^3$

- Wasserstoff oder
- eine verzweigte oder unverzweigte $C_1$-$C_6$-Alkylgruppe, insbesondere eine $C_1$-$C_4$-Alkylgruppe wie vorstehend genannt;

$R^4$

- Wasserstoff,
- Halogen wie vorstehend genannt, insbesondere Fluor und Chlor, oder
- eine verzweigte oder unverzweigte $C_1$-$C_6$-Alkylgruppe, insbesondere eine $C_1$-$C_4$-Alkylgruppe wie vorstehend genannt;

$R^5$

- Wasserstoff
- eine verzweigte oder unverzweigte $C_1$-$C_6$-Alkylgruppe, insbesondere eine $C_1$-$C_4$-Alkylgruppe wie vorstehend genannt; oder
- $R^3$ und $R^4$, $R^4$ und $R^5$ oder bevorzugt $R^3$ und $R^5$ bilden zusammen eine verzweigte oder unverzweigte $C_2$-$C_4$-Alkylen- oder $C_2$-$C_4$-Alkenylenkette wie Ethylen, Propylen, 2-Methylpropylen, Butylen, Prop-2-enylen und But-2-enylen, insbesondere Propylen, Butylen und But-2-enylen;

$R^6$

- Wasserstoff;
- eine verzweigte oder unverzweigte $C_1$-$C_6$-Alkylgruppe, insbesondere eine $C_1$-$C_4$-Alkylgruppe wie vorstehend genannt;
- eine partiell oder vollständig halogenierte $C_1$-$C_4$-Alkylgruppe, wie vorstehend genannt, insbesondere Difluormethyl und Trifluormethyl, wobei die Alkylgruppe noch einen Phenylrest tragen kann; bevorzugt ist 2,2,2-Trifluor-1-phenylethyl;
- eine $C_2$-$C_6$-Alkenylgruppe wie vorstehend genannt, insbesondere Ethenyl und Prop-2-enyl;
- eine $C_1$-$C_4$-Alkoxy-$C_1$-$C_4$-alkylgruppe wie vorstehend genannt, insbesondere Methoxymethyl, Methoxyethyl, Ethoxymethyl und Ethoxyethyl;
- eine $C_1$-$C_4$-Alkylthio-$C_1$-$C_4$-alkylgruppe wie Methylthiomethyl, Methylthioethyl, Ethylthiomethyl und Ethylthioethyl;
- eine $C_3$-$C_7$-Cycloalkyl- oder $C_5$-$C_7$-Cycloalkenylgruppe wie vorstehend genannt, insbesondere Cyclopentyl, Cyclohexyl und Cyclohexenyl, die beide noch einen bis drei der folgenden Reste tragen können: Hydroxyl, $C_1$-$C_4$-Alkyl wie vorstehend genannt, partiell oder vollständig halogeniertes $C_1$-$C_4$-Alkyl wie vorstehend genannt, insbesondere Trifluormethyl, $C_1$-$C_4$-Alkoxy wie vorstehend genannt, insbesondere Methoxy und Ethoxy, $C_1$-$C_4$-Alkoxy-$C_1$-$C_4$-alkyl wie vorstehend genannt, insbesondere Methoxymethyl und Ethoxymethyl oder $C_1$-$C_4$-Alkylthio-$C_1$-$C_4$-alkyl wie vorstehend genannt, insbesondere Methylthiomethyl;
- die Phenyl- oder die Pydridylgruppe, die beide noch einen bis drei der folgenden Reste tragen können: Nitro, Cyano, Hydroxyl, Halogen wie vorstehend genannt, insbesondere Fluor und Chlor, $C_1$-$C_4$-Alkyl wie vorstehend genannt, partiell oder vollständig halogeniertes $C_1$-$C_4$-Alkyl wie vorstehend genannt, insbesondere Trifluormethyl, $C_1$-$C_4$-Alkoxy wie vorstehend genannt, insbesondere Methoxy und Ethoxy, partiell oder vollständig halogeniertes $C_1$-$C_4$-Alkoxy wie vorstehend genannt, insbesondere Trifluormethoxy, $C_1$-$C_4$-Alkylthio wie vorstehend genannt, insbesondere Methylthio, $C_1$-$C_4$-Alkoxy-$C_1$-$C_4$-alkyl wie vorstehend genannt, insbesondere Methoxymethyl und Ethoxymethyl oder $C_1$-$C_4$-Alkylthio-$C_1$-$C_4$-alkyl wie vorstehend genannt, insbesondere Methylthiomethyl; besonders bevorzugt ist Phenyl, Pyridyl, 4-Fluorphenyl und 4-Chlorphenyl.

Mögliche Verbindungen I sind beispielhaft in den folgenden Tabellen A und B aufgeführt:

Tabelle A

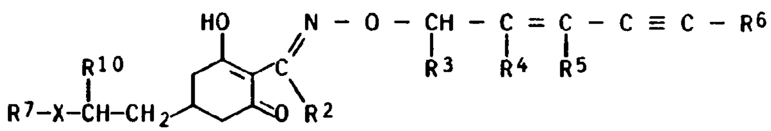

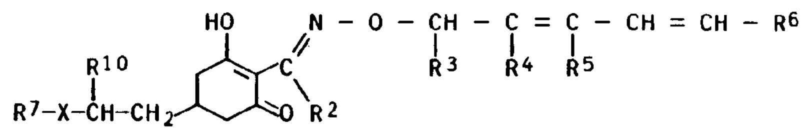

(I) (R1 = $-CH_2-CH(R10)-X-R7$ mit R10 = H; $CH_3$)

| R10 | X | R7 | R2 | R3 | R5 | R4 | R6 |
|---|---|---|---|---|---|---|---|
| H | S | 4-$CF_3$-phenyl | Ethyl | H | H | F | 4-F-phenyl |
| H | S | 4-F-phenyl | Propyl | $CH_3$ | H | F | 4-Cl-phenyl |
| $CH_3$ | S | 4-($CF_3$-O-)phenyl | Ethyl | H | H | H | Phenyl |
| $CH_3$ | S | 4-Br-phenyl | Propyl | $CH_3$ | H | H | Pyridin-3-yl |
| H | S | 4-$CHF_2$-phenyl | Ethyl | $-(CH_2)_3-$ | | H | 4-F-phenyl |
| H | S | 4-($CHF_2$-O-)phenyl | Propyl | H | $CH_3$ | $CH_3$ | 3-Cl-phenyl |
| $CH_3$ | S | 4-($CHF_2$-$CF_2$-O-)phenyl | Ethyl | H | H | F | 3-$CF_3$-phenyl |
| $CH_3$ | S | 2-F-4-$CF_3$-phenyl | Propyl | $C_2H_5$ | H | F | 4-$CH_3$-O-phenyl |
| H | S | 5-$CF_3$-pyridin-2-yl | Ethyl | $CH_3$ | $CH_3$ | H | tert.-Butyl |
| H | S | 5-$CHF_2$-pyridin-2-yl | Propyl | $-(CH_2)_3-$ | | F | 2,2,2-$F_3$-1-phenylethyl |
| $CH_3$ | S | 5-$CF_3$-pyridin-2-yl | Ethyl | n-$C_3H_7$ | H | F | 3,3,3-$F_3$-prop-2-yl |
| $CH_3$ | S | 5-F-pyridin-2-yl | Propyl | H | $CH_3$ | F | 2-$CH_3$O-prop-2-yl |
| H | S | 5-($CF_3$-O-)pyridin-2-yl | Ethyl | $CH_3$ | H | H | Cyclohexyl |
| H | S | 5-($CHF_2$-O-)-pyridin-2-yl | Propyl | $CH_3$ | H | Et | Cyclopentyl |
| $CH_3$ | S | 4-Cl-phenyl | Ethyl | $-(CH_2)_3-$ | | $CH_3$ | Propen-2-yl |
| $CH_3$ | S | 5-Br-pyridin-2-yl | Propyl | H | H | F | 3-$CF_3$-phenyl |
| $CH_3$ | S | 4-$CF_3$-phenyl | Ethyl | $C_2H_5$ | H | F | H |
| H | S | 4-$CF_3$-phenyl | Ethyl | H | H | F | 3-$CH_3$-pent-3-yl |
| H | S | 4-F-phenyl | Propyl | $CH_3$ | H | F | Cyclohex-2-en-1-yl |

Tabelle A (Fortsetzung)

| $R^{10}$ | X | $R^7$ | $R^2$ | $R^3$ | $R^5$ | $R^4$ | $R^6$ |
|---|---|---|---|---|---|---|---|
| $CH_3$ | S | 4-($CF_3$-O-)phenyl | Ethyl | H | H | H | Pyridin-2-yl |
| $CH_3$ | S | 4-Br-phenyl | Propyl | $CH_3$ | H | H | Trimethylsilyl |
| H | S | 4-$CHF_2$-phenyl | Ethyl | -$(CH_2)_3$- | | H | Isopropyl |
| H | S | 4-($CHF_2$-O-)phenyl | Propyl | H | $CH_3$ | $CH_3$ | n-Hexyl |
| $CH_3$ | S | 4-($CHF_2$-$CF_2$-O-)phenyl | Ethyl | H | H | F | 4-$CH_3O$-phenyl |
| $CH_3$ | S | 2-F-4-$CF_3$-phenyl | Propyl | $C_2H_5$ | H | F | 4-$CF_3$-phenyl |
| H | S | 5-$CF_3$-pyridin-2-yl | Ethyl | $CH_3$ | $CH_3$ | H | 3,4-$Cl_2$-phenyl |
| H | S | 5-$CHF_2$-pyridin-2-yl | Propyl | -$(CH_2)_3$- | | F | 4-Cl-phenyl |
| $CH_3$ | S | 5-$CF_3$-pyridin-2-yl | Ethyl | n-$C_3H_7$ | H | F | 3-F-phenyl |
| $CH_3$ | S | 5-F-pyridin-2-yl | Propyl | H | $CH_3$ | F | Phenyl |
| H | S | 5-($CF_3$-O-)pyridin-2-yl | Ethyl | $CH_3$ | H | H | H |
| H | S | 5-($CHF_2$-O-)-pyridin-2-yl | Propyl | $CH_3$ | H | Et | tert.-Butyl |
| $CH_3$ | S | 4-Cl-phenyl | Ethyl | -$(CH_2)_3$- | | $CH_3$ | 1,1,1-$F_3$-prop-2-yl |
| $CH_3$ | S | 5-Br-pyridin-2-yl | Propyl | H | H | F | 2-$CH_2Cl$-but-2-yl |
| $CH_3$ | S | 4-$CF_3$-phenyl | Ethyl | $C_2H_5$ | H | F | 4-F-phenyl |
| H | SO | 4-$CF_3$-phenyl | Ethyl | H | H | F | 4-F-phenyl |
| H | SO | 4-F-phenyl | Propyl | $CH_3$ | H | F | 4-Cl-phenyl |
| $CH_3$ | SO | 4-($CF_3$-O-)phenyl | Ethyl | H | H | H | Phenyl |
| $CH_3$ | SO | 4-Br-phenyl | Propyl | $CH_3$ | H | H | Pyridin-3-yl |
| H | SO | 4-$CHF_2$-phenyl | Ethyl | -$(CH_2)_3$- | | H | 4-F-phenyl |
| H | SO | 4-($CHF_2$-O-)phenyl | Propyl | H | $CH_3$ | $CH_3$ | 3-Cl-phenyl |
| $CH_3$ | SO | 4-($CHF_2$-$CF_2$-O-)phenyl | Ethyl | H | H | F | 3-$CF_3$-phenyl |
| $CH_3$ | SO | 2-F-4-$CF_3$-phenyl | Propyl | $C_2H_5$ | H | F | 3-$CH_3O$-phenyl |

Tabelle A (Fortsetzung)

| $R^{10}$ | X | $R^7$ | $R^2$ | $R^3$ | $R^5$ | $R^4$ | $R^6$ |
|---|---|---|---|---|---|---|---|
| H | SO | 5-$CF_3$-pyridin-2-yl | Ethyl | $CH_3$ | $CH_3$ | H | tert.-Butyl |
| H | SO | 5-$CHF_2$-pyridin-2-yl | Propyl | $-(CH_2)_3-$ | | F | 2,2,2-$F_3$-1-phenylethyl |
| $CH_3$ | SO | 5-$CF_3$-pyridin-2-yl | Ethyl | n-$C_3H_7$ | H | F | 3,3,3-$F_3$-prop-2-yl |
| $CH_3$ | SO | 5-F-pyridin-2-yl | Propyl | H | $CH_3$ | F | 2-$CH_3O$-prop-2-yl |
| H | SO | 5-($CF_3$-O-)pyridin-2-yl | Ethyl | $CH_3$ | H | H | Cyclohexyl |
| H | SO | 5-($CHF_2$-O-)-pyridin-2-yl | Propyl | $CH_3$ | H | Et | Cyclopentyl |
| $CH_3$ | SO | 4-Cl-phenyl | Ethyl | $-(CH_2)_3-$ | | $CH_3$ | Propen-2-yl |
| $CH_3$ | SO | 5-Br-pyridin-2-yl | Propyl | H | H | F | 3-$CF_3$-phenyl |
| $CH_3$ | SO | 4-$CF_3$-phenyl | Ethyl | $C_2H_5$ | H | F | H |
| H | $SO_2$ | 4-$CF_3$-phenyl | Ethyl | H | H | F | 3-$CH_3$-pent-3-yl |
| H | $SO_2$ | 4-F-phenyl | Propyl | $CH_3$ | H | F | Cyclohex-2-en-1-yl |
| $CH_3$ | $SO_2$ | 4-($CF_3$-O-)phenyl | Ethyl | H | H | H | Pyridin-2-yl |
| $CH_3$ | $SO_2$ | 4-Br-phenyl | Propyl | $CH_3$ | H | H | Trimethylsilyl |
| H | $SO_2$ | 4-$CHF_2$-phenyl | Ethyl | $-(CH_2)_3-$ | | H | Isopropyl |
| H | $SO_2$ | 4-($CHF_2$-O-)phenyl | Propyl | H | $CH_3$ | $CH_3$ | n-Hexyl |
| $CH_3$ | $SO_2$ | 4-($CHF_2$-$CF_2$-O-)phenyl | Ethyl | H | H | F | 4-$CH_3O$-phenyl |
| $CH_3$ | $SO_2$ | 2-F-4-$CF_3$-phenyl | Propyl | $C_2H_5$ | H | F | 4-$CF_3$-phenyl |
| H | $SO_2$ | 5-$CF_3$-pyridin-2-yl | Ethyl | $CH_3$ | $CH_3$ | H | 3,4-$Cl_2$-phenyl |
| H | $SO_2$ | 5-$CHF_2$-pyridin-2-yl | Propyl | $-(CH_2)_3-$ | | F | 4-Cl-phenyl |
| $CH_3$ | $SO_2$ | 5-$CF_3$-pyridin-2-yl | Ethyl | n-$C_3H_7$ | H | F | 3-F-phenyl |
| $CH_3$ | $SO_2$ | 5-F-pyridin-2-yl | Propyl | H | $CH_3$ | F | Phenyl |
| H | $SO_2$ | 5-($CF_3$-O-)pyridin-2-yl | Ethyl | $CH_3$ | H | H | H |
| H | $SO_2$ | 5-($CHF_2$-O-)-pyridin-2-yl | Propyl | $CH_3$ | H | Et | tert.-Butyl |

Tabelle A (Fortsetzung)

| $R^{10}$ | X | $R^7$ | $R^2$ | $R^3$ | $R^5$ | $R^4$ | $R^6$ |
|---|---|---|---|---|---|---|---|
| $CH_3$ | $SO_2$ | 4-Cl-phenyl | Ethyl | $-(CH_2)_3-$ | | $CH_3$ | 1,1,1-$F_3$-prop-2-yl |
| $CH_3$ | $SO_2$ | 5-Br-pyridin-2-yl | Propyl | H | H | F | 2-$CH_2Cl$-but-2-yl |
| $CH_3$ | $SO_2$ | 4-$CF_3$-phenyl | Ethyl | $C_2H_5$ | H | F | 4-F-phenyl |
| H | S | Ethyl | Ethyl | H | H | F | 4-F-phenyl |
| H | S | Ethyl | Propyl | $CH_3$ | H | F | 4-Cl-phenyl |
| $CH_3$ | S | Ethyl | Ethyl | H | H | H | Phenyl |
| $CH_3$ | S | Ethyl | Propyl | $CH_3$ | H | H | Pyridin-3-yl |
| H | S | Ethyl | Ethyl | $-(CH_2)_3-$ | | H | 4-F-phenyl |
| H | S | Ethyl | Propyl | H | $CH_3$ | $CH_3$ | 3-Cl-phenyl |
| $CH_3$ | S | Ethyl | Ethyl | H | H | F | 3-$CF_3$-phenyl |
| $CH_3$ | S | Ethyl | Propyl | $C_2H_5$ | H | F | 4-$CH_3O$-phenyl |
| H | S | Ethyl | Ethyl | $CH_3$ | $CH_3$ | H | tert.-Butyl |
| H | S | Ethyl | Propyl | $-(CH_2)_3-$ | | F | 2,2,2-$F_3$-1-phenylethyl |
| $CH_3$ | S | Ethyl | Ethyl | Propyl | H | F | 1,1,1-$F_3$-prop-2-yl |
| $CH_3$ | S | Ethyl | Propyl | H | $CH_3$ | F | 2-$CH_3O$-prop-2-yl |
| H | S | Ethyl | Ethyl | $CH_3$ | H | H | Cyclohexyl |
| H | S | Ethyl | Propyl | $CH_3$ | H | Et | Cyclopentyl |
| $CH_3$ | S | Ethyl | Ethyl | $-(CH_2)_3-$ | | $CH_3$ | Propen-2-yl |
| $CH_3$ | S | Ethyl | Propyl | H | H | F | 3-$CF_3$-phenyl |
| $CH_3$ | S | Ethyl | Ethyl | $C_2H_5$ | H | F | H |

Tabelle B

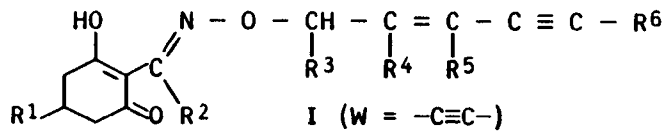
I (W = $-C\equiv C-$)

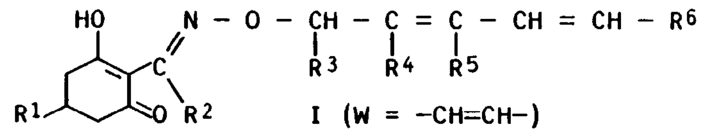
I (W = $-CH=CH-$)

| $R^1$ | $R^2$ | $R^3$ | $R^5$ | $R^4$ | $R^6$ |
|---|---|---|---|---|---|
| 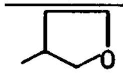 | $CH_2CH_3$ | H | H | F | Phenyl |
| 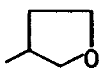 | $(CH_2)_2CH_3$ | $CH_3$ | H | F | tert.-Butyl |
| 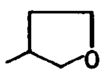 | $CH_2CH_3$ | $C_2H_5$ | H | H | n-Hexyl |
| 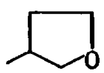 | $(CH_2)_2CH_3$ | H | $CH_3$ | F | 4-F-phenyl |
| 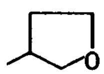 | $CH_2CH_3$ | $-(CH_2)_3-$ | | F | 2,2,2-$F_3$-1-phenylethyl |
| 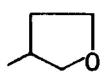 | $(CH_2)_2CH_3$ | $CH_3$ | $CH_3$ | H | 1-$CH_3O$-eth-1-yl |

Tabelle B (Fortsetzung)

| $R^1$ | $R^2$ | $R^3$ | $R^5$ | $R^4$ | $R^6$ |
|---|---|---|---|---|---|
| 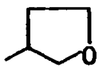 | $CH_2CH_3$ | n-$C_3H_7$ | H | F | Pyridin-3-yl |
| 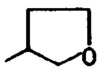 | $(CH_2)_2CH_3$ | H | H | F | Cyclohexyl |
| 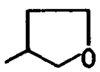 | $CH_2CH_3$ | -$(CH_2)_3$- | | H | 4-Cl-phenyl |
| 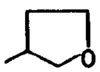 | $(CH_2)_2CH_3$ | H | $CH_3$ | F | 3-$CH_3$-pent-3-yl |
| 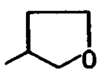 | $CH_2CH_3$ | H | H | H | Cyclohex-2-en-1-yl |
| 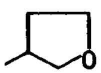 | $(CH_2)_2CH_3$ | $CH_3$ | $CH_3$ | F | Phenyl |
| 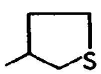 | $CH_2CH_3$ | H | H | F | Phenyl |
| 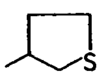 | $(CH_2)_2CH_3$ | $CH_3$ | H | F | tert.-Butyl |

Tabelle B (Fortsetzung)

| $R^1$ | $R^2$ | $R^3$ | $R^5$ | $R^4$ | $R^6$ |
|---|---|---|---|---|---|
| 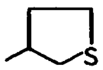 | $CH_2CH_3$ | $C_2H_5$ | H | H | n-Hexyl |
| 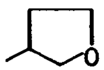 | $(CH_2)_2CH_3$ | H | $CH_3$ | F | p-F-phenyl |
| 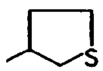 | $CH_2CH_3$ | $-(CH_2)_3-$ | | F | 2,2,2-$F_3$-1-phenylethyl |
| 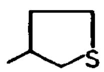 | $(CH_2)_2CH_3$ | $CH_3$ | $CH_3$ | H | 1-$CH_3O$-eth-1-yl |
| 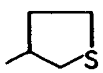 | $CH_2CH_3$ | n-$C_3H_7$ | H | F | Pyridin-3-yl |
| 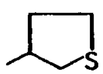 | $(CH_2)_2CH_3$ | H | H | F | Cyclohexyl |
| 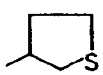 | $CH_2CH_3$ | $-(CH_2)_3-$ | | H | 4-Cl-phenyl |
| 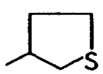 | $(CH_2)_2CH_3$ | H | $CH_3$ | F | 3-$CH_3$-pent-3-yl |

Tabelle B (Fortsetzung)

| $R^1$ | $R^2$ | $R^3$ | $R^5$ | $R^4$ | $R^6$ |
|---|---|---|---|---|---|
| 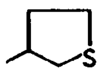 | $CH_2CH_3$ | H | H | H | Cyclohex-2-en-1-yl |
| 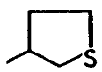 | $(CH_2)_2CH_3$ | $CH_3$ | $CH_3$ | F | Phenyl |
| 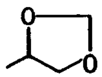 | $CH_2CH_3$ | $CH_3$ | $CH_3$ | F | 1-Cl-tert.-butyl |
| 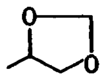 | $(CH_2)_2CH_3$ | $CH_3$ | H | F | Cyclopentyl |
| 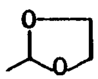 | $CH_2CH_3$ | H | H | F | Phenyl |
| 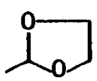 | $(CH_2)_2CH_3$ | $CH_3$ | H | F | tert.-Butyl |
| 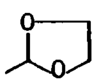 | $CH_2CH_3$ | $C_2H_5$ | H | H | n-Hexyl |
| 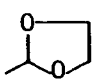 | $(CH_2)_2CH_3$ | H | $CH_3$ | F | p-F-phenyl |

Tabelle B (Fortsetzung)

| $R^1$ | $R^2$ | $R^3$ | $R^5$ | $R^4$ | $R^6$ |
|---|---|---|---|---|---|
| 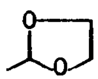 | $CH_2CH_3$ | $-(CH_2)_3-$ | | F | 2,2,2-$F_3$-1-phenylethyl |
| 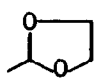 | $(CH_2)_2CH_3$ | $CH_3$ | $CH_3$ | H | 1-$CH_3O$-eth-1-yl |
| 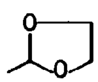 | $CH_2CH_3$ | n-$C_3H_7$ | H | F | Pyridin-3-yl |
| 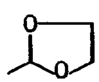 | $(CH_2)_2CH_3$ | H | H | F | Cyclohexyl |
| 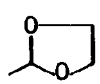 | $CH_2CH_3$ | $-(CH_2)_3-$ | | H | p-Cl-phenyl |
| 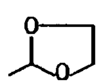 | $(CH_2)_2CH_3$ | H | $CH_3$ | F | 3-$CH_3$-pent-3-yl |
| 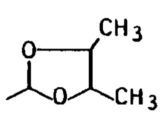 | $CH_2CH_3$ | $CH_3$ | $CH_3$ | F | Cyclohex-2-en-1-yl |
| 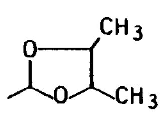 | $(CH_2)_2CH_3$ | H | H | H | Phenyl |

Tabelle B (Fortsetzung)

| $R^1$ | $R^2$ | $R^3$ | $R^5$ | $R^4$ | $R^6$ |
|---|---|---|---|---|---|
| 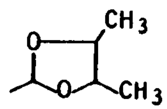 | $CH_2CH_3$ | $C_2H_5$ | $CH_3$ | F | 1-Cl-tert.-Butyl |
| 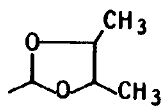 | $(CH_2)_2CH_3$ | H | H | F | Phenyl |
| 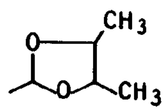 | $CH_2CH_3$ | $CH_3$ | H | F | tert.-Butyl |
| 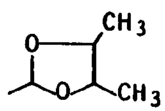 | $(CH_2)_2CH_3$ | $C_2H_5$ | H | H | n-Hexyl |
| 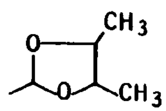 | $CH_2CH_3$ | H | $CH_3$ | F | p-F-phenyl |
| 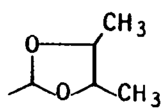 | $(CH_2)_2CH_3$ | $-(CH_2)_3-$ | | F | 1,1,1-$F_3$-2-phenylethyl |
| 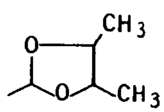 | $CH_2CH_3$ | $CH_3$ | $CH_3$ | H | 1-$CH_3O$-eth-1-yl |

Tabelle B (Fortsetzung)

| $R^1$ | $R^2$ | $R^3$ | $R^5$ | $R^4$ | $R^6$ |
|---|---|---|---|---|---|
| 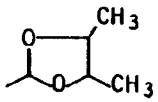 | $(CH_2)_2CH_3$ | $n-C_3H_7$ | H | F | Pyridin-3-yl |
| 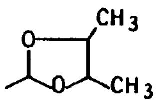 | $CH_2CH_3$ | H | H | F | Cyclohexyl |
| 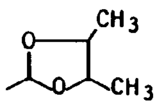 | $(CH_2)_2CH_3$ | $-(CH_2)_3-$ | | F | 4-Cl-phenyl |
| 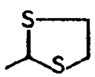 | $CH_2CH_3$ | H | $CH_3$ | F | 3-$CH_3$-pent-3-yl |
| 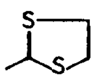 | $(CH_2)_2CH_3$ | H | H | H | Cyclohex-2-en-1-yl |
| 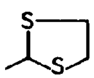 | $CH_2CH_3$ | $CH_3$ | $CH_3$ | F | Phenyl |
| 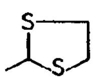 | $(CH_2)_2CH_3$ | H | H | F | Phenyl |
| 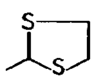 | $CH_2CH_3$ | $CH_3$ | H | F | tert.-Butyl |

Tabelle B (Fortsetzung)

| $R^1$ | $R^2$ | $R^3$ | $R^5$ | $R^4$ | $R^6$ |
|---|---|---|---|---|---|
| 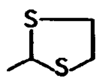 | $(CH_2)_2CH_3$ | $C_2H_5$ | H | H | n-Hexyl |
| 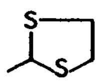 | $CH_2CH_3$ | H | $CH_3$ | F | 4-F-phenyl |
| 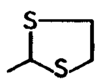 | $(CH_2)_2CH_3$ | $-(CH_2)_3-$ | | F | 2,2,2-$F_3$-1-phenylethyl |
| 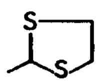 | $CH_2CH_3$ | $CH_3$ | $CH_3$ | H | 1-$CH_3O$-eth-1-yl |
| 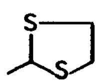 | $(CH_2)_2CH_3$ | n-$C_3H_7$ | H | F | Pyridin-3-yl |
| 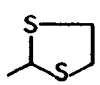 | $CH_2CH_3$ | H | H | $CH_3$ | Cyclohexyl |
| 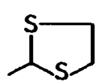 | $(CH_2)_2CH_3$ | $-(CH_2)_3-$ | | H | 4-Cl-phenyl |
| 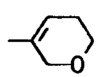 | $CH_2CH_3$ | H | $CH_3$ | F | 3-$CH_3$-pent-3-yl |

Tabelle B (Fortsetzung)

| $R^1$ | $R^2$ | $R^3$ | $R^5$ | $R^4$ | $R^6$ |
|---|---|---|---|---|---|
| 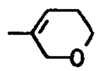 | $(CH_2)_2CH_3$ | H | H | $CH_3$ | Cyclohex-2-en-1-yl |
| 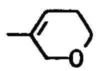 | $CH_2CH_3$ | $CH_3$ | $CH_3$ | F | Phenyl |
| 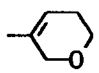 | $(CH_2)_2CH_3$ | $C_2H_5$ | $CH_3$ | F | 1-Cl-tert.-butyl |
| 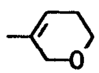 | $CH_2CH_3$ | H | H | F | Phenyl |
| 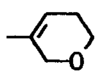 | $(CH_2)_2CH_3$ | $CH_3$ | H | F | tert.-Butyl |
| 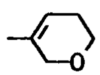 | $CH_2CH_3$ | $C_2H_5$ | H | H | n-Hexyl |
| 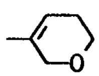 | $(CH_2)_2CH_3$ | H | $CH_3$ | F | 4-F-phenyl |
| 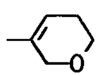 | $CH_2CH_3$ | $-(CH_2)_3-$ | | F | 2,2,2-$F_3$-1-phenylethyl |

Tabelle B (Fortsetzung)

| $R^1$ | $R^2$ | $R^3$ | $R^5$ | $R^4$ | $R^6$ |
|---|---|---|---|---|---|
| 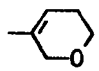 | $(CH_2)_2CH_3$ | $CH_3$ | $CH_3$ | H | 1-$CH_3$O-eth-1-yl |
| 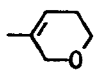 | $CH_2CH_3$ | n-$C_3H_5$ | H | F | Pyridin-3-yl |
| 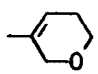 | $(CH_2)_2CH_3$ | H | H | F | Cyclohexyl |
| 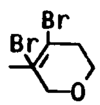 | $CH_2CH_3$ | $-(CH_2)_3-$ | | H | 4-Cl-phenyl |
| 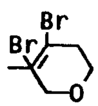 | $(CH_2)_2CH_3$ | H | $CH_3$ | F | 3-$CH_3$-pent-3-yl |
| 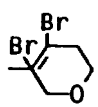 | $CH_2CH_3$ | H | H | H | Cyclohex-2-en-1-yl |
| 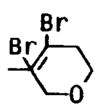 | $(CH_2)_2CH_3$ | $CH_3$ | $CH_3$ | F | Phenyl |

Tabelle B (Fortsetzung)

| $R^1$ | $R^2$ | $R^3$ | $R^4$ | $R^5$ | $R^6$ |
|---|---|---|---|---|---|
| 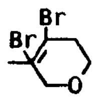 | $CH_2CH_3$ | $C_2H_5$ | $CH_3$ | F | Cyclopentyl |
| 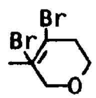 | $(CH_2)_2CH_3$ | H | H | F | Phenyl |
| 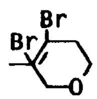 | $CH_2CH_3$ | $CH_3$ | H | F | tert.-Butyl |
| 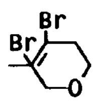 | $(CH_2)_2CH_3$ | $C_2H_5$ | H | H | n-Hexyl |
| 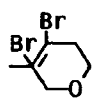 | $CH_2CH_3$ | H | $CH_3$ | F | p-F-phenyl |
| 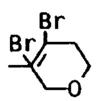 | $(CH_2)_2CH_3$ | $-(CH_2)_3-$ | | F | 2,2,2-$F_3$-1-phenylethyl |
| 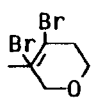 | $CH_2CH_3$ | $CH_3$ | $CH_3$ | H | 1-$CH_3O$-eth-1-yl |

Tabelle B (Fortsetzung)

| R1 | R2 | R3 | R5 | R4 | R6 |
|---|---|---|---|---|---|
| 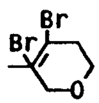 | $(CH_2)_2CH_3$ | $n-C_3H_7$ | H | F | Pyridin-3-yl |
| 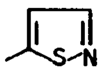 | $CH_2CH_3$ | H | F | H | Cyclohexyl |
| 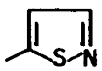 | $(CH_2)_2CH_3$ | $-(CH_2)_3-$ | | H | 4-Cl-phenyl |
| 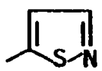 | $CH_2CH_3$ | H | $CH_3$ | F | 3-$CH_3$-pent-3-yl |
| 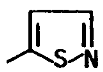 | $(CH_2)_2CH_3$ | H | H | $CH_3$ | Cyclohex-2-en-1-yl |
| 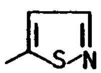 | $CH_2CH_3$ | $CH_3$ | $CH_3$ | $CH_3$ | Phenyl |
| 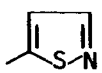 | $(CH_2)_2CH_3$ | H | H | F | Phenyl |
| 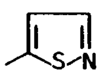 | $CH_2CH_3$ | $CH_3$ | H | F | tert.-Butyl |

Tabelle B (Fortsetzung)

| $R^1$ | $R^2$ | $R^3$ | $R^5$ | $R^4$ | $R^6$ |
|---|---|---|---|---|---|
| 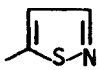 | $(CH_2)_2CH_3$ | $C_2H_5$ | H | H | n-Hexyl |
| 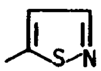 | $CH_2CH_3$ | H | $CH_3$ | F | p-F-phenyl |
| 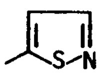 | $(CH_2)_2CH_3$ | $-(CH_2)_3-$ | | F | 2,2,2-$F_3$-1-phenylethyl |
| 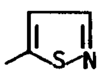 | $CH_2CH_3$ | $CH_3$ | $CH_3$ | H | Isopropyl |
| 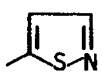 | $(CH_2)_2CH_3$ | n-$C_3H_7$ | H | F | Pyridin-3-yl |
| 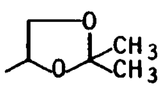 | $CH_2CH_3$ | H | H | F | Cyclohexyl |
| 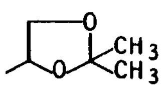 | $(CH_2)_2CH_3$ | $-(CH_2)_3-$ | | H | 4-Cl-Phenyl |
| 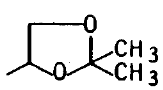 | $CH_2CH_3$ | H | $CH_3$ | F | 3-$CH_3$-pent-3-yl |

Tabelle B (Fortsetzung)

| $R^1$ | $R^2$ | $R^3$ | $R^5$ | $R^4$ | $R^6$ |
|---|---|---|---|---|---|
| 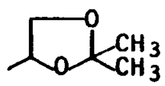 | $(CH_2)_2CH_3$ | H | H | H | Cyclohex-2-en-1-yl |
| 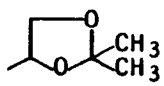 | $CH_2CH_3$ | $CH_3$ | $CH_3$ | F | Phenyl |
| 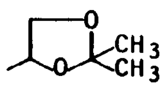 | $(CH_2)_2CH_3$ | $C_2H_5$ | $CH_3$ | F | 1-Cl-tert.-Butyl |
| 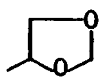 | $CH_2CH_3$ | $CH_3$ | H | F | Cyclopentyl |
| 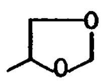 | $(CH_2)_2CH_3$ | H | H | F | Phenyl |
| 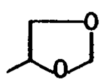 | $CH_2CH_3$ | $CH_3$ | H | F | tert.-Butyl |
| 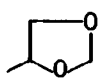 | $(CH_2)_2CH_3$ | $C_2H_5$ | H | H | n-Hexyl |
| 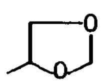 | $CH_2CH_3$ | H | $CH_3$ | F | p-F-phenyl |

Tabelle B (Fortsetzung)

| $R^1$ | $R^2$ | $R^3$ | $R^5$ | $R^4$ | $R^6$ |
|---|---|---|---|---|---|
| 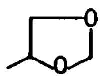 | $(CH_2)_2CH_3$ | $-(CH_2)_3-$ | | F | 2,2,2-$F_3$-1-phenylethyl |
| 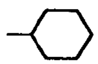 | $CH_2CH_3$ | $CH_3$ | $CH_3$ | H | 1-$CH_3O$-eth-1-yl |
| 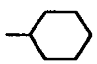 | $(CH_2)_2CH_3$ | n-$C_3H_7$ | H | F | Pyridin-3-yl |
| 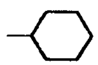 | $CH_2CH_3$ | H | H | F | Cyclohexyl |
| 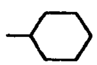 | $(CH_2)_2CH_3$ | $-(CH_2)_3-$ | | H | 4-Cl-phenyl |
| 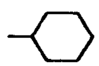 | $CH_2CH_3$ | H | $CH_3$ | F | 3-$CH_3$-pent-3-yl |
| 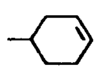 | $(CH_2)_2CH_3$ | H | H | H | Cyclohex-2-en-1-yl |

Tabelle B (Fortsetzung)

| $R^1$ | $R^2$ | $R^3$ | $R^5$ | $R^4$ | $R^6$ |
|---|---|---|---|---|---|
| 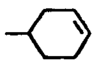 | $CH_2CH_3$ | $CH_3$ | $CH_3$ | F | Phenyl |
| 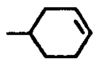 | $(CH_2)_2CH_3$ | $C_2H_5$ | $CH_3$ | F | 1-Cl-tert.-Butyl |
| 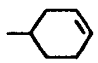 | $CH_2CH_3$ | $CH_3$ | H | F | Cyclopentyl |
| 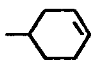 | $(CH_2)_2CH_3$ | H | $CH_3$ | $CH_3$ | p-F-Phenyl |
| 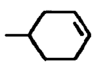 | $CH_2CH_3$ | $CH_3$ | $CH_3$ | F | p-Cl-Phenyl |
| 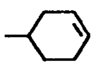 | $(CH_2)_2CH_3$ | H | H | H | tert.-Butyl |
| 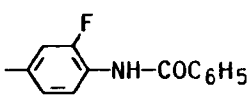 | $(CH_2)_2CH_3$ | H | H | F | Phenyl |
| 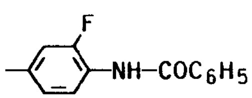 | $CH_2CH_3$ | $CH_3$ | H | F | tert.-Butyl |

Tabelle B (Fortsetzung)

| $R^1$ | $R^2$ | $R^3$ | $R^5$ | $R^4$ | $R^6$ |
|---|---|---|---|---|---|
| 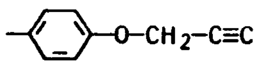 | $(CH_2)_2CH_3$ | $C_2H_5$ | H | H | n-Hexyl |
| 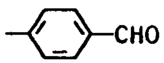 | $CH_2CH_3$ | H | $CH_3$ | F | p-F-phenyl |
| 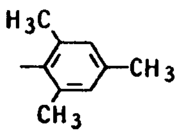 | $(CH_2)_2CH_3$ | $-(CH_2)_3-$ | | F | 2,2,2-$F_3$-1-phenylethyl |
| 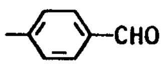 | $CH_2CH_3$ | $CH_3$ | $CH_3$ | H | 1-$CH_3O$-eth-1-yl |
| 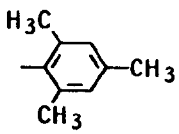 | $(CH_2)_2CH_3$ | n-$C_3H_7$ | H | F | Pyridin-3-yl |
| 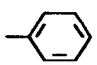 | $CH_2CH_3$ | H | H | F | Cyclohexyl |
| 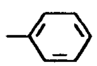 | $(CH_2)_2CH_3$ | $-(CH_2)_3-$ | | H | p-Cl-phenyl |

Tabelle B (Fortsetzung)

| $R^1$ | $R^2$ | $R^3$ | $R^5$ | $R^4$ | $R^6$ |
|---|---|---|---|---|---|
| 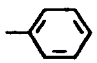 | $CH_2CH_3$ | H | $CH_3$ | F | 3-$CH_3$-pent-3-yl |
| 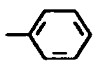 | $(CH_2)_2CH_3$ | H | H | H | Cyclohex-2-en-1-yl |
| 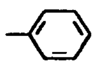 | $CH_2CH_3$ | $CH_3$ | $CH_3$ | F | Phenyl |
| 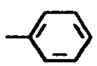 | $(CH_2)_2CH_3$ | $C_2H_5$ | $CH_3$ | F | 1-Cl-tert.-Butyl |
| 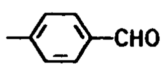 | $CH_2CH_3$ | $CH_3$ | H | F | Cyclopentyl |
| 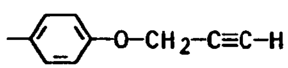 | $(CH_2)_2CH_3$ | H | $CH_3$ | F | 4-F-phenyl |

Ungesättigte Cyclohexenonoximether I, bei denen $R^3$ Wasserstoff, $R^4$ Wasserstoff oder Fluor, $R^5$ Wasserstoff oder Methyl und $R^6$ Wasserstoff, Methyl oder durch Halogen substituiertes Phenyl bedeuten, sind ganz besonderes bevorzugt.

Die Verbindungen I können bei der Herstellung als E-/Z-Isomerengemische anfallen, wobei sich die Isomeren durch die cis- oder trans-Stellung der Substituenten an der Doppelbindung bzw. an den Doppelbindungen im Aminether-Teil unterscheiden. Die Isomeren können gewünschtenfalls nach den hierfür üblichen Methoden, z.B. durch Kristallisation oder Chromatographie, getrennt werden.

Die ungesättigten Cyclohexenonoximether I sind auf verschiedene Weise erhältlich, und zwar vorzugsweise durch Umsetzung von alkylcarbonylsubstituierten Cyclohexandionen II mit Hydroxylaminen III

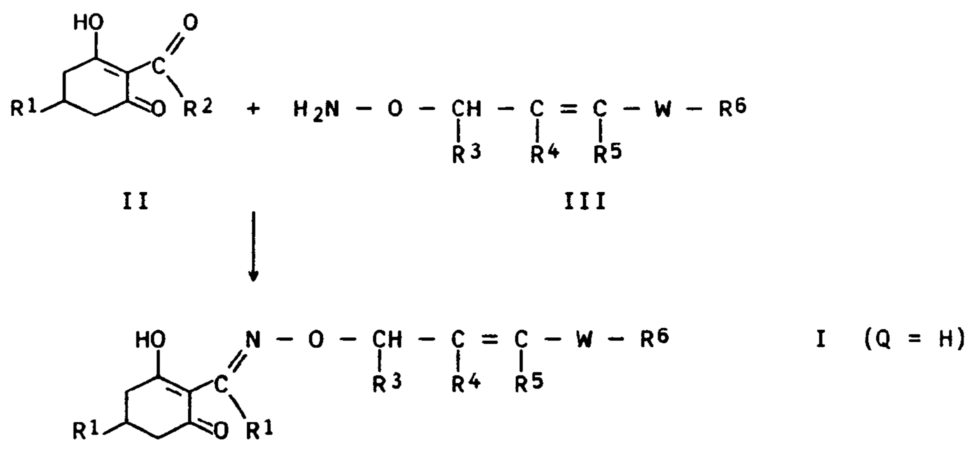

Die Umsetzung erfolgt in an sich bekannter Weise (vgl. EP-A 169 521) in einem inerten Lösungs- oder Verdünnungsmittel in Gegenwart einer Base.

Als Lösungsmittel kommen Dimethylsulfoxid, Alkohole wie Methanol, Ethanol, Isopropanol und Cyclohexanol, aliphatische Kohlenwasserstoffe wie n-Hexan und Cyclohexan, aromatische Kohlenwasserstoffe wie Toluol und o-, m-, p-Xylol, chlorierte Kohlenwasserstoffe wie Methylenchlorid und 1,2-Dichlorethan, Ester wie Essigsäuremethylester, Nitrile wie Acetonitril, cyclische Ether wie Dioxan und Tetrahydrofuran sowie Gemische der genannten Solvention in Betracht. Je nach verwendeten Lösungsmittel erhält man ein ein- oder zweiphasiges Reaktionsgemisch.

Als Basen eignen sich beispielsweise Alkalimetall- und Erdalkalimetallcarbonate wie Natriumcarbonat, Kaliumcarbonat und Calciumcarbonat, Alkalimetall- und Erdalkalimetallhydrogencarbonate wie Natriumhydrogencarbonat und Kaliumhydrogencarbonat, Alkalimetall- und Erdalkalimetalloxide wie Natriumoxid, Kaliumoxid, Calciumoxid und Magnesiumoxid, Alkalimetall- und Erdalkalimetallacetate wie Natriumacetat, Kaliumacetat und Calciumacetat, Alkalimetall- und Erdalkalimetallhydroxide, insbesondere Natriumhydroxid und Kaliumhydroxid, Alkalimetall- und Erdalkalimetallalkoholate, insbesondere Natriummethanolat, Kaliummethanolat, Natriumethanolat und Kaliumethanolat sowie Amine wie Triethylamin, Pyridin und 4-Dimethylaminopyridin, und zwar für eine vollständige Umsetzung in mindestens stöchiometrische Menge, bezogen auf die Menge an II.

Vorzugsweise führt man die Reaktion in Methanol mit Natriumhydrogencarbonat als Base durch, wobei die Menge an Base in der Regel zwischen 0,5 und 2 mol-%, bezogen auf die Menge an III, liegt.

Das Hydroxylamin III kann als freie Base, beispielsweise gelöst in Wasser, oder bevorzugt in Form eines geeigneten Ammoniumsalzes eingesetzt werden.

Die Mengenverhältnisse sind nicht kritisch. Normalerweise werden die Edukte II und III in stöchiometrischem Verhältnis eingesetzt, jedoch kann auch ein Überschuß der Verbindung III, etwa bis zu 20 mol-%, vorteilhaft sein. Verwendet man das Hydroxylamin III gleichzeitig als Base, so liegt es in einem größeren Überschuß vor.

Im allgemeinen arbeitet man unter Normaldruck oder unter dem Eigendruck des jeweiligen Lösungsmittels, wobei sich eine Reaktionstemperatur zwischen 0°C und dem Siedepunkt des Lösungsmittels, insbesondere zwischen 20 und 80°C, empfiehlt.

Die Aufarbeitung des Reaktionsgemisches erfolgt nach an sich bekannten Methoden, beispielsweise durch Entfernen des Lösungsmittels, Verteilen des Rückstandes in Methylenchlorid/Wasser und Isolieren des Produktes der organischen Phase.

Die alkylcarbonylsubstituierten Cyclohexandione II sind aus den Druckschriften EP-A 80 301, EP-A 125

094, EP-A 137 174, EP-A 177 913, EP-A 142 741 und US-A 4 249 937 bekannt oder können nach an sich bekannten Methoden, beispielsweise durch Umsetzung von Cyclohexan-1,3-dionen IV mit Säurechloriden V unter anschließender Umlagerung mit bestimmten Imidazol- oder Pydridinderivaten (vgl. JP-A 79/063052) erhalten werden:

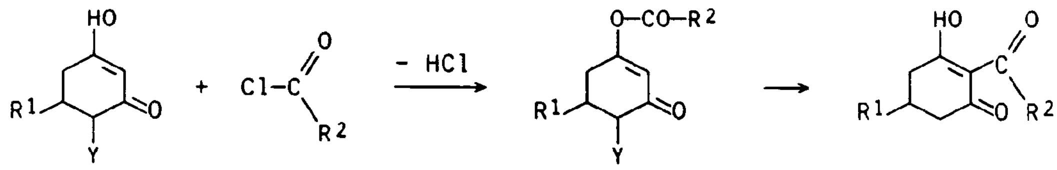


IV V II

Y = Wasserstoff oder Methoxycarbonyl.

Eine weitere Synthesemöglichkeit zur Herstellung der alkylsubstituierten Cyclohexandione II aus den Cyclohexan-1,3-dionen IV wird in Tetrahedron Lett., 2491 (1975) beschrieben.

Die Hydroxylamine IIIa

$$H_2N - O - \underset{R^3}{\underset{|}{CH}} - \underset{R^4}{\underset{|}{C}} = \underset{R^5}{\underset{|}{C}} - C \equiv C - R^6 \qquad \text{IIIa,}$$

die eine Doppel- und eine Dreifachbindung enthalten, sind aus EP-A 341 048 und EP-A 361 827 bekannt oder nach den dort beschriebenen Methoden herstellbar.

Die Hydroxylamine IIIb

$$H_2N - O - \underset{R^3}{\underset{|}{CH}} - \underset{R^4}{\underset{|}{C}} = \underset{R^5}{\underset{|}{C}} - CH = CH - R^6 \qquad \text{IIIb,}$$

die zwei Doppelbindungen enthalten, sind neu. Sie können nach an sich bekannten Methoden erhalten werden, beispielsweise durch Umsetzung von Vinylmagnesiumchlorid mit Zimtaldehyden VI, Behandlung des Verfahrensproduktes VII mit wäßriger Bromwasserstoffsäure [vgl. Chem. Reviews 56, 753 (1956)], Überführung der erhaltenen bromierten Dienverbindung VIII in ein Phthalimid IX und anschließender Freisetzung des Hydroxylamins [vgl. EP-A 244 786]:

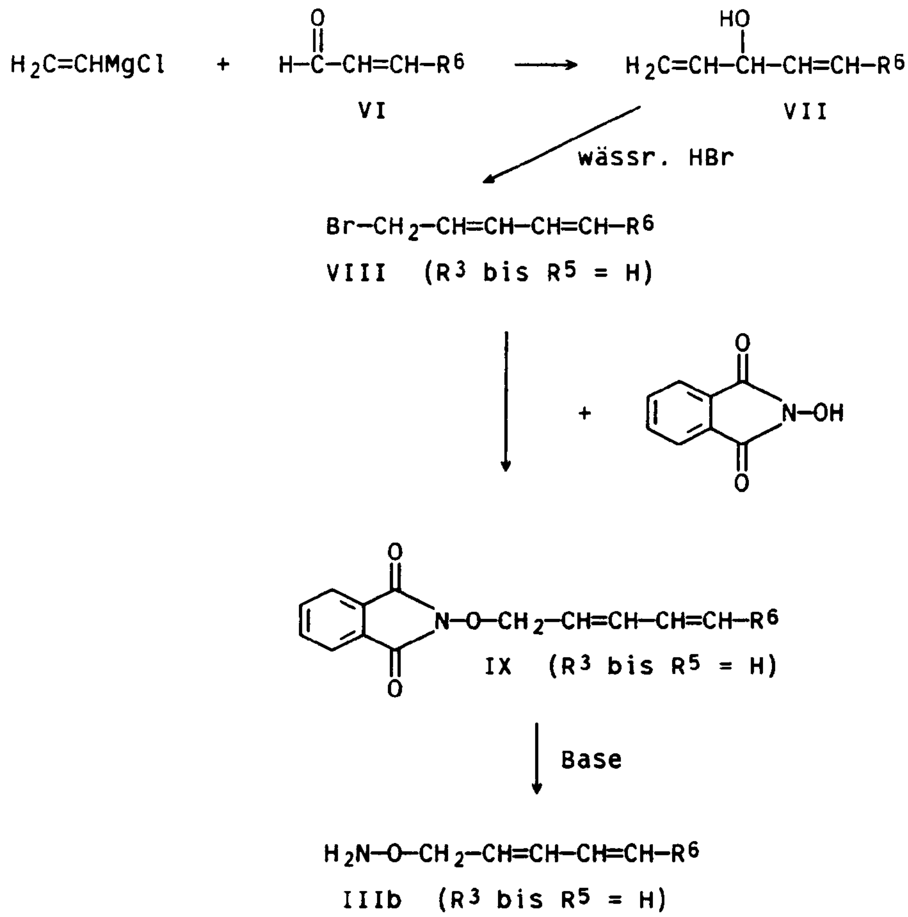


Auch die bromierten Diene der Formel VIII sowie die Phthalimide der Formel IX

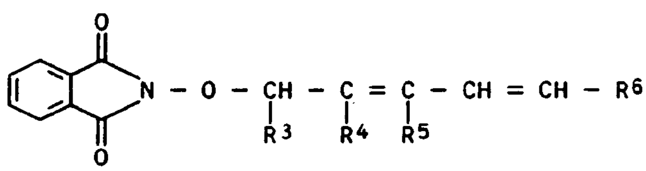
IX

sind neu.

Durch Umsetzung mit Alkalimetallsalzen, beispielsweise Alkalimetallhydroxiden wie Natrium- und Kaliumhydroxid und Alkalimetallalkoholaten wie Natrium- und Kaliummethanolat, in wäßriger Lösung oder in einem aprotischen organischen Lösungsmittel wie Methanol, Ethanol, Aceton, Toluol und o-, m-, p-Xylol können die ungesättigten Cyclohexenonoximether I in ihre Alkalimetallsalze überführt werden. Weitere Salze der Verbindungen I, beispielsweise Erdalkalimetallsalze wie Magnesium-, Calcium- und Bariumsalze, Übergangsmetallsalze wie Mangan-, Eisen-, Kupfer- und Zinksalze, Ammoniumsalze mit einem bis vier $C_1$-$C_4$-Alkyl-, Phenyl- und/oder Benzylsubstituenten wie Diisopropylammonium-, Tetramethylammonium-, Tetrabutylammonium und Trimethylbenzylammoniumsalze, Phosphoniumsalze und Sulfoniumsalze, insbesondere Trialkylsulfoniumsalze, sind aus den Alkalimetallsalzen, insbesondere den Natriumsalzen der Verbindungen I erhältlich.

Ungesättigte Cyclohexenonoximether I, wobei Q eine $C_1$-$C_6$-Alkylcarbonylgruppe oder die Benzoylgruppe bedeutet, sind durch an sich bekannte Veresterungsreaktionen von Verbindungen I, wobei Q Wasserstoff bedeutet, mit $C_1$-$C_6$-Alkancarbonsäuren oder mit Benzoesäure herstellbar.

Sowohl die Isomerengemische als auch die reinen Isomeren der ungesättigten Cyclohexenonderivate I eignen sich als Herbizide, insbesondere zur Bekämpfung von Pflanzenarten aus der Familie der Gramineen (Gräser). Im allgemeinen sind sie verträglich und somit selektiv in breitblättrigen Kulturen sowie in monokotylen (einkeimblättrigen) Gewächsen, welche nicht zu den Gramineen zählen. Einigen Derivate der Verbindungen I können jedoch auch Selektivität in Gramineen aufweisen, wodurch sich unverwünschte Gräser gezielt bekämpfen lassen.

Die ungesättigten Cyclohexenonoximether I bzw. die sie enthaltenden herbiziden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel, wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten im allgemeinen zwischen 0,01 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Beispiele für solche Zubereitungen sind:

I. eine Lösung aus 90 Gew.-Teilen der Verbindung Nr. 1.01 und 10 Gew.-Teilen N-Methyl-α-pyrrolidon, die zur Anwendung in Form kleinster Tropfen geeignet ist;

II. eine Mischung aus 20 Gew.-Teilen der Verbindung Nr. 1.02, 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.-Teilen des Anlagerungsproduktes und 40 Mol Ethylenoxid an 1 Mol Ricinusöl. Durch feines Verteilen des Gemisches in 100 000 Gew.-Teilen Wasser erhält man eine Dispersion, die 0,02 Gew.-% des Wirkstoffs enthält.

III. eine wäßrige Dispersion aus 20 Gew.-Teilen der Verbindung Nr. 1.03, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl. Die Mischung dieser Dispersion mit 100 000 Gewichtsteilen Wasser enthält 0,02 Gew.-% des Wirkstoffes.

IV. eine wäßrige Dispersion aus 20 Gew.-Teilen der Verbindung Nr. 1.04, 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 mol Ethylenoxid an 1 mol Ricinusöl. Die Mischung dieser Dispersion mit 100 000 Gew.-Teilen Wasser enthält 0,02 % des Wirkstoffes;

V. eine in einer Hammermühle vermahlene Mischung aus 80 Gew.-Teilen der Verbindung Nr. 1.05, 3 Gew.-

Teilen des Natriumsalzes der Diisobutylnaphtalin-α-sulfonsäure, 10 Gew.-Teilen des Natriumsalzes einer Ligninsulfonsäure aus einer Sulfitablauge und 7 Gew.-Teilen pulverförmigem Kieselsäuregel. Durch feines Verteilen der Mischung in 20 000 Gew.-Teilen Wasser erhält man eine Spritzbrühe, die 0,1 Gew.-% des Wirkstoffs enthält;

VI. eine innige Mischung aus 3 Gew.-Teilen der Verbindung Nr. 1.06 und 97 Gew.-Teilen feinteiligem Kaolin. Dieses Stäubemittel enthält 3 Gew.-% Wirkstoff;

VII. eine innige Mischung aus 30 Gew.-Teilen der Verbindung Nr. 1.07, 92 Gew.-Teilen pulverförmigem Kieselsäuregel und 8 Gew.-Teilen Paraffinöl, das auf die Oberfläche dieses Kieselsäuregels gesprüht wurde. Diese Aufbereitung gibt dem Wirkstoff eine gute Haftfähigkeit;

VIII. eine stabile wäßrige Dispersion aus 40 Gew.-Teilen der Verbindung Nr. 1.08, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;

IX. eine stabile ölige Dispersion aus 20 Gew.-Teilen der Verbindung Nr. 1.09, 2 Gew.-Teilen des Calciumsalzes der Dodecylbenzolsulfonsäure, 8 Gew.-Teilen Fettalkohol-polyglykolether, 20 Gew.- Teilen des Natriumsalzes eines Phenolsulfonsäure-harnstoffformaldehyd-Kondensates und 68 Gew.-Teilen eines paraffinischen Mineralöls;

X. eine in einer Hammermühle vermahlene Mischung aus 10 Gew.-Teilen der Verbindung Nr. 1.10, 4 Gew.-Teilen des Natriumsalzes der Diisobutylnaphthalin-α-sulfonsäure, 20 Gew.-Teilen des Natriumsalzes einer Ligninsulfonsäure aus einer Sulfitablauge, 38 Gew.- Teilen Kieselsäuregel und 38 Gew.-Teilen Kaolin. Durch feines Verteilen der Mischung in 10 000 Gew.-Teilen Wasser erhält man eine Spritzbrühe, die 0,1 Gew.% des Wirkstoffs enthält.

Die Applikation der herbiziden Mittel bzw. der Wirkstoffe kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Bekämpfungsziel, Jahreszeit, Zielpflanzen und Wachstumsstadium 0,001 bis 3,0, vorzugsweise 0,01 bis 1 kg/ha aktive Substanz (a.S.).

In Anbetracht der Vielseitigkeit der Applikationsmethoden können die ungesättigten Cyclohexenonoximether bzw. sie enthaltende Mittel noch in einer weiteren Zahl von Kulturplfanzen zur Beseitigung unerwünschter Pflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Beta vulgaris spp. altissima | Zuckerrübe |
| Beta vulgaris spp. rapa | Futterrübe |
| Brassica napus var. napus | Raps |
| Brassica napus var. napobrassica | Kohlrübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus sinensis | Apfelsine, Orange |
| Coffea arabica (Coffea canephora, Coffea liberica) | Kaffee |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum, Gossypium vitifolium) | Baumwolle |
| Helianthus annuus | Sonnenblume |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lens culinaris | Linse |
| Linum usitatissimum | Faserlein |
| Lycopersicon lycopersicum | Tomate |
| Malus spp. | Apfel |
| Manihot esculenta | Maniok |
| Medicago sativa | Luzerne |
| Musa spp. | Obst- und Mehlbanane |
| Nicotiana tabacum (N. rustica) | Tabak |
| Olea europaea | Ölbaum |
| Oryza sativa | Reis |
| Phaseolus lunatus | Mondbohne |
| Phaseolus vulgaris | Buschbohnen |

| Botanischer Name | Deutscher Name |
|---|---|
| Picea abies | Rotfichte |
| Pinus spp. | Kiefer |
| Pisum sativum | Gartenerbse |
| Prunus avium | Süßkirsche |
| Prunus persica | Pfirsich |
| Pyrus communis | Birne |
| Ribes sylvestre | Rote Johannisbeere |
| Ricinus communis | Rizinus |
| Saccharum officinarum | Zuckerrohr |
| Secale cereale | Roggen |
| Solanum tuberosum | Kartoffel |
| Sorghum bicolor (s. vulgare) | Mohrenhirse |
| Theobroma cacao | Kakaobaum |
| Trifolium pratense | Rotklee |
| Triticum aestivum | Weizen |
| Triticum durum | Hartweizen |
| Vicia faba | Pferdebohnen |
| Vitis vinifera | Weinrebe |
| Zea mays | Mais |

Zur Verbreiterung des Wirkungsspektrums und zur Erzielung synergistischer Effekte können die ungesättigten Cyclohexenonoximether I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Imidazolinone, Sulfonamide, Sulfonylharnstoffe, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs-und Spurenelementmängeln eingesetzt werden. Es können auch nichtphytotoxische Öle und Ölkonzentrate zugesetzt werden.

Herstellungsbeispiele

Beispiel 1

2-[(Z)-3-Methyl-pent-3-en-1-in-5-yloxy-iminobutyl]-3-hydroxy-5-(2-H-tetrahydropyran-4-yl)-2-cyclohexen-1-on

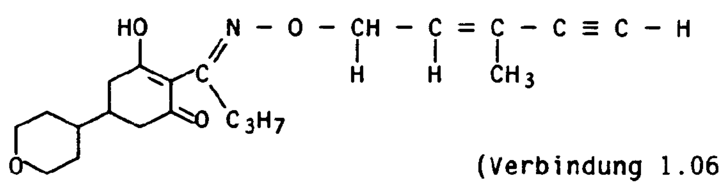

(Verbindung 1.06)

Eine Mischung aus 3 g (11 mmol) 2-Butyryl-3-hydroxy-5-(2H-tetrahydropyran-4-yl)-cyclohex-2-en-1-on, 1,5 g (13 mmol) (Z)-5-Aminooxy-3-methylpent-3-en-1-in und 100 ml Methanol wurde 16 Stunden bei 25°C gerührt. Nach Entfernen des Lösungsmittels nahm man den Rückstand in 100 ml 10 gew.-%iger wäßriger Natronlauge auf. Die wäßrige Phase wurde dreimal mit je 100 ml Methylenchlorid extrahiert, danach unter Eiskühlung mit konzentrierter Salzsäure angesäuert (pH = 1) und dreimal mit je 100 ml Diethylether extrahiert. Die vereinigten organischen Phasen wurden wie üblich auf das Produkt hin aufgearbeitet. Die Reinigung des Rohproduktes erfolgte mittels Chromatographie an Kieselgel (Essigester als Laufmittel).
Ausbeute: 73 %;
300 MHz-$^1$H-NMR (in $CDCl_3$, TMS als Standard): 1,95 ppm (s,3H); 3,20 (s,1H); 4,75 (d,2H); 5,90 (t,1H).

Vorstufe 1α:

N-(Z)-(3-Methylpent-3-en-1-in-5-yloxy)-phthalimid

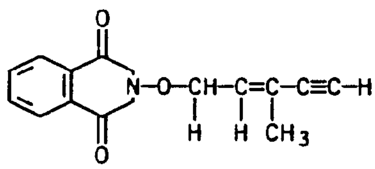

(Zwischenprodukt 3.01)

Zu einer Lösung von 40,0 g (0,4 mol) 5-Hydroxy-3-methylpent-3-en-1-in in 960 ml wasserfreiem Tetrahydrofuran wurden 71,8 g (0,44 mol) N-Hydroxyphthalimid und 115,4 g (0,44 mol) Triphenylphosphin gegeben. Zu dieser Mischung wurden innerhalb von 3 Stunden 85,2 g (0,44 mol) Diethylazodicarboxylat so zugetropft, daß die Temperatur 35°C nicht überstieg. Anschließend rührte man das Reaktionsgemisch über Nacht, entfernte dann das Lösungsmittel unter reduziertem Druck und versetzte den Rückstand mit 400 ml Toluol. Nach Entfernen von unlöslichen Anteilen wurde die organische Phase zweimal mit 5 gew.-%iger wäßriger Natronlauge und einmal mit gesättigter wäßriger Natriumchloridlösung gewaschen und anschließend wie üblich auf das Produkt hin aufgearbeitet. Die Reinigung erfolgte mittels Chromatographie an Kieselgel N 60 (Laufmittel: Toluol) und anschließender Umkristallisation aus Isopropanol.
Ausbeute: 48 %; Fp.: 102-103°C.
250 MHz-$^1$H-NMR (in $d^6$-DMSO): 1,85 ppm (s,3H); 4,24 ppm (s,1H); 4,82 ppm (d,2H); 6,08 ppm (t,1H); 7,88 ppm (s,4H).

Vorstufe 1β:

(Z)-5-Aminooxy-3-methylpent-3-en-1-in

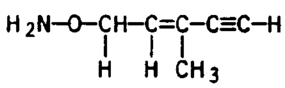

(Zwischenprodukt 2.04)

Eine Mischung aus 45 g (0,19 mol) N-(Z)-(3-Methylpent-3-en-1-in-5-yloxy)-phthalimid in 95 ml (1,57 mol) Ethanolamin wurde 4 Stunden bei etwa 20°C gerührt und dann in 300 ml eisgekühlte, gesättigte Natriumchloridlösung gegossen. Aus der wäßrigen Phase extrahiert man das Produkt dreimal mit je 100 ml Dichlormethan. Die vereinigten organischen Phasen wurden mit gesättigter wäßriger Natriumchloridlösung gewaschen und über Magnesiumsulfat getrocknet. Nach Entfernen des Lösungsmittels unter reduziertem Druck erhielt man das Produkt als Öl.
Ausbeute: 63 %
250 $MH_2$-$^1$H-NMR (in $CDCl_3$, TMS als Standard): 1,93 ppm (s,3H); 3,2 ppm (s,1H); 4,38 ppm (d,2H); 5,4 ppm (breit,2H); 5,9 ppm (t,1H).

Beispiel 2

E,E-N-1-Aminooxy-5-(4-fluorphenyl)-2,4-pentadienyloxy)phthalimid

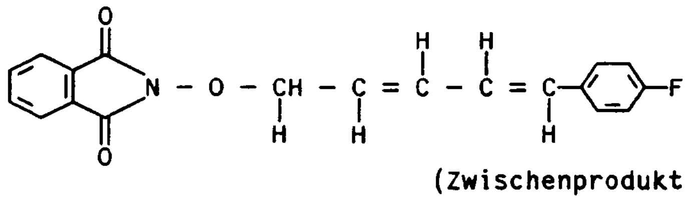

(Zwischenprodukt 3.02)

Zu einer Mischung aus 10,4 g (43 mmol) 1-Brom-5-(4-fluorphenyl)-2,4-pentadien, 9,8 g (60 mmol) N-Hydroxyphthalimid und 45 ml N-Methylpyrrolidon wurden bei etwa 20°C 8,3 ml (60 mmol) Triethylamin getropft. Nach 24 Stunden Rühren goß man die Mischung in 200 ml Wasser und extrahierte mehrfach mit Essigsäureethylester, wonach die vereinigten organischen Phasen mit 5 gew.-%iger wäßriger Natronlauge und dann mit gesättigter wäßriger Natriumchloridlösung gewaschen wurden. Nach Trocknen mit Magnesiumsulfat und Entfernen des Lösungsmittels wurde der Rückstand in 70 ml Ethanol umkristallisiert. Ausbeute: 57 %. Fp.: 155-157°C.

Vorstufe 2α:

1-Brom-5-(4-fluorphenyl)-2,4-pentadien

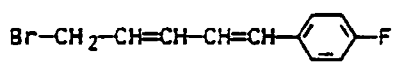

Zu 20 g (0,11 mol) 1-(4-Fluorphenyl)-penta-1,4-dien-3-ol wurden nacheinander 40 ml 47 gew.%ige wäßrige Bromwasserstoffsäure und 80 ml Toluol gegeben. Nach 10 Minuten trennte man die Phasen, wonach die organische Phase mit gesättigter wäßriger Natriumhydrogencarbonatlösung gewaschen, mit Natriumsulfat getrocknet und unter reduziertem Druck eingeengt wurde.
Ausbeute: 18,6 g (Rohprodukt).

Vorstufe 2β:

1-(4-Fluorphenyl)-penta-1,4-dien-3-ol

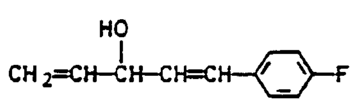

Zu einer Lösung von 75 g (0,5 mol) 4-Fluorzimtaldehyd in 100 ml Tetrahydrofuran wurden bei 0 bis 5°C 350 ml einer 1,6 molaren Lösung von Vinylmagnesiumchlorid (≙ 0,55 mol Vinylmagnesiumchlorid) getropft. Nach 4 Stunden Rühren bei 25°C hydrolysierte man bei 0 bis 5°C mit 150 ml gesättigter wäßriger Ammoniumchloridlösung, trennte dann die Phasen und trocknete die organische Phase mit Natriumsulfat. Nach Entfernen des Lösungsmittels bei reduziertem Druck wurde der Rückstand im Vakuum destilliert. Ausbeute: 41 g; Kp.0,2 = 92°C.

In der folgenden Tabelle 1 sind noch weitere Verbindungen I aufgeführt, die auf die gleiche Weise hergestellt wurden oder herstellbar sind. Die Tabellen 2 und 3 enthalten weitere Zwischenprodukte III und IX.

Tabelle 1

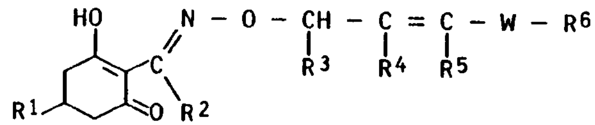

(I)

| Nr. | $R^1$ | $R^2$ | W | $R^3$ | $R^4$ | $R^5$ | $R^6$ | Konfig. | physik. Daten (300 MHz-$^1$H-NMR [ppm] *)) |
|---|---|---|---|---|---|---|---|---|---|
| 1.01 | 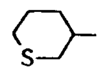 | Ethyl | -C≡C- | H | H | $CH_3$ | H | Z | 1,95 (s,3H), 3,20 (s,1H), 4,75 (d,2H), 5,90 (t,1H) |
| 1.02 | 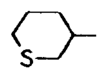 | Propyl | -C≡C- | H | H | $CH_3$ | H | Z | 1,95 (s,3H), 3,20 (s,1H), 4,75 (d,2H), 5,90 (t,1H) |
| 1.03 | 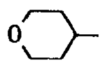 | Ethyl | -C≡C- | H | H | $CH_3$ | H | Z | 1,95 (s,3H), 3,20 (s,1H), 4,75 (d,2H), 5,90 (t,1H) |
| 1.04 | 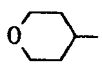 | Propyl | -C≡C- | H | H | $CH_3$ | H | Z | 1,95 (s,3H), 3,20 (s,1H), 4,75 (d,2H), 5,90 (t,1H) |
| 1.05 | 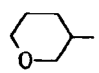 | Ethyl | -C≡C- | H | H | $CH_3$ | H | Z | 1,95 (s,3H), 3,20 (s,1H), 4,75 (d,2H), 5,90 (t,1H) |
| 1.06 | 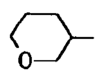 | Propyl | -C≡C- | H | H | $CH_3$ | H | Z | 1,95 (s,3H), 3,20 (s,1H), 4,75 (d,2H), 5,90 (t,1H) |
| 1.07 | 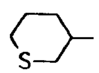 | Ethyl | E-CH=CH- | H | H | H | 4-F-phenyl | E | 1,15 (t,3H), 4,62 (d,2H), 5,95 (dt,1H) |
| 1.08 | 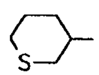 | Propyl | E-CH=CH- | H | H | H | 4-F-phenyl | E | 0,95 (t,3H), 4,6 (d,2H), 5,95 (dt,1H) |

*) in $CDCl_3$, TMS als interner Standard

Tabelle 1 (Fortsetzung)

| Nr. | R1 | R2 | W | R3 | R4 | R5 | R6 | Konfig. | physik. Daten (300 MHz-$^1$H-NMR [ppm] *)) |
|---|---|---|---|---|---|---|---|---|---|
| 1.09 | 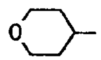 | Ethyl | E-CH=CH- | H | H | H | 4-F-phenyl | E | 1,15 (t,3H), 4,62 (d,2H), 5,95 (dt,1H) |
| 1.10 | 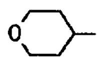 | Propyl | E-CH=CH- | H | H | H | 4-F-phenyl | E | 0,95 (t,3H), 4,62 (d,2H), 5,95 (dt,1H) |
| 1.11 | 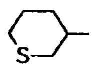 | Ethyl | $-C\equiv C-$ | H | H | $CH_3$ | H | E | |
| 1.12 | 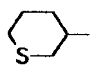 | Propyl | $-C\equiv C-$ | H | H | $CH_3$ | H | E | |
| 1.13 | 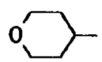 | Ethyl | $-C\equiv C-$ | H | H | $CH_3$ | H | E | |
| 1.14 | 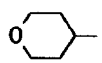 | Propyl | $-C\equiv C-$ | H | H | $CH_3$ | H | E | |
| 1.15 | 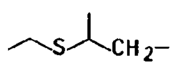 | Propyl | $-C\equiv C-$ | H | H | $CH_3$ | H | E | |
| 1.16 | 2,4,6-$(CH_3)_3$-Cyclohexyl | Ethyl | $-C\equiv C-$ | H | H | $CH_3$ | H | E | |
| 1.17 | 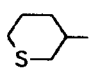 | Ethyl | $-C\equiv C-$ | H | H | $CH_3$ | 4-Cl-phenyl | Z | 1,15 (t), 2,0 (s), 4,8 (d), 5,9 (t) |
| 1.18 | 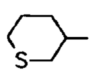 | Propyl | $-C\equiv C-$ | H | H | $CH_3$ | 4-Cl-phenyl | Z | 0,95 (t), 2,0 (s), 4,8 (d), 5,9 (t) |
| 1.19 | 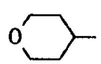 | Ethyl | $-C\equiv C-$ | H | H | $CH_3$ | 4-Cl-phenyl | Z | 1,15 (t), 2,0 (s), 4,8 (d), 5,9 (t) |

Tabelle 1 (Fortsetzung)

| Nr. | R1 | R2 | W | R3 | R4 | R5 | R6 | Konfig. | physik. Daten (300 MHz-$^1$H-NMR [ppm] *)) |
|---|---|---|---|---|---|---|---|---|---|
| 1.20 | 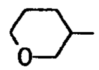 | Propyl | -C≡C- | H | H | $CH_3$ | 4-Cl-phenyl | Z | 0,95 (t), 2,0 (s), 4,8 (d), 5,9 (t) |
| 1.21 | 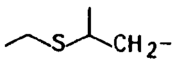 | Propyl | -C≡C- | H | H | $CH_3$ | 4-Cl-phenyl | Z | 0,95 (t), 2,0 (s), 4,8 (d), 5,9 (t) |
| 1.22 | 2,4,6-$(CH_3)_3$-phenyl | Ethyl | -C≡C- | H | H | $CH_3$ | 4-Cl-phenyl | Z | 1,2 (t), 2,0 (s), 4,8 (d), 5,95 (t) |
| 1.23 | 2,4,6-$(CH_3)_3$-phenyl | Ethyl | -C≡C- | H | H | $CH_3$ | H | E | 1,15 (t), 1,9 (s), 4,65 (d), 6,1 (t) |
| 1.24 | 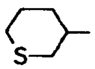 | Ethyl | -C≡C- | H | H | $CH_3$ | 4-F-phenyl | E | 1,1 (t), 2,0 (s), 4,8 (d), 5,9 (t) |
| 1.25 | 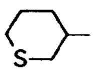 | Propyl | -C≡C- | H | H | $CH_3$ | 4-F-phenyl | E | 0,95 (t), 2,0 (s), 4,8 (d), 5,9 (t) |
| 1.26 | 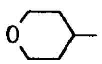 | Propyl | -C≡C- | H | H | $CH_3$ | 4-F-phenyl | E | 0,95 (t), 2,0 (s), 4,8 (d), 5,9 (t) |
| 1.27 | 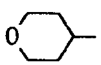 | Ethyl | -C≡C- | H | H | $CH_3$ | 4-F-phenyl | Z | 1,15 (t), 2,0 (s), 3,35 (t), 4,8 (d), 7,0 (m) |
| 1.28 | 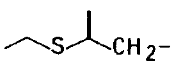 | Propyl | -C≡C- | H | H | $CH_3$ | 4-F-phenyl | Z | 0,95 (t), 2,0 (s), 4,8 (d), 7,05 (m) |
| 1.29 | 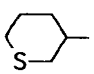 | Propyl | -C≡C- | H | H | $CH_3$ | 1-OH-2,2,6,6-$(CH_3)_4$-Cyclohexyl | Z | 0,95 (t), 1,1 (s), 1,15 (s), 4,75 (d), 5,8 (m) |

Tabelle 1 (Fortsetzung)

| Nr. | R1 | R2 | W | R3 | R4 | R5 | R6 | Konfig. | physik. Daten (300 MHz-1H-NMR [ppm] *)) |
|---|---|---|---|---|---|---|---|---|---|
| 1.30 | 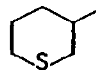 | Ethyl | -C≡C- | H | H | $CH_3$ | 1-OH-2,2,6,6-$(CH_3)_4$-Cyclohexyl | Z | 1,1 (s), 1,15 (s), 4,75 (d), 5,8 (m) |
| 1.31 | 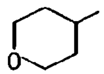 | Ethyl | -C≡C- | H | H | $CH_3$ | 1-OH-2,2,6,6-$(CH_3)_4$-Cyclohexyl | Z | 1,1 (s), 1,15 (s), 3,35 (t), 4,7 (d) |
| 1.32 | 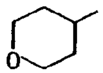 | Propyl | -C≡C- | H | H | $CH_3$ | 1-OH-2,2,6,6-$(CH_3)_4$-Cyclohexyl | Z | 0,95 (s), 1,1 (s), 1,15 (s), 3,35 (t), 4,7 (d) |
| 1.33 | 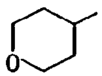 | Ethyl | -C≡C- | H | H | $CH_3$ | 1-OH-cyclohexyl | Z | 1,15 (t), 1,9 (s), 3,35 (t), 4,7 (d), 5,8 (m) |
| 1.34 | 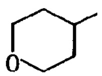 | Propyl | -C≡C- | H | H | $CH_3$ | 1-OH-cyclohexyl | Z | 0,95 (t), 1,9 (s), 3,35 (t), 4,7 (d), 5,8 (m) |
| 1.35 | 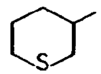 | Propyl | -C≡C- | H | H | $CH_3$ | 1-OH-cyclohexyl | Z | 0,95 (t), 1,9 (s), 4,7 (d), 5,8 (m) |
| 1.36 | 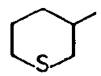 | Ethyl | E-C=C- | H | H | H | Ethyl | E | 1,0 (t), 1,15 (t), 4,55 (d), 5,85 (m) |
| 1.37 | 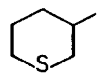 | Propyl | E-C=C- | H | H | H | Ethyl | E | 0,95 (t), 1,0 (t), 4,55 (d), 5,85 (m) |
| 1.38 | 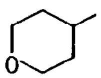 | Ethyl | E-C=C- | H | H | H | Ethyl | E | 1,0 (t), 1,15 (t), 3,35 (t), 4,55 (d), 5,85 (m) |

Tabelle 1 (Fortsetzung)

| Nr. | $R^1$ | $R^2$ | W | $R^3$ | $R^4$ | $R^5$ | $R^6$ | Konfig. | physik. Daten (300 MHz-$^1$H-NMR [ppm] *)) |
|---|---|---|---|---|---|---|---|---|---|
| 1.39 | 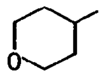 | Propyl | E-C=C- | H | H | H | Ethyl | E | 0,95 (t), 1,0 (t), 3,35 (t), 4,55 (d), 5,85 (m) |
| 1.40 | 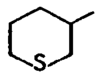 | Ethyl | E-C=C- | H | H | H | Methyl | E | |
| 1.41 | 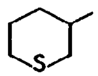 | Propyl | E-C=C- | H | H | H | Methyl | E | |
| 1.42 | 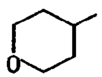 | Ethyl | E-C=C- | H | H | H | Methyl | E | |
| 1.43 | 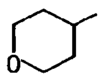 | Propyl | E-C=C- | H | H | H | Methyl | E | |
| 1.44 | 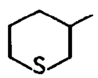 | Ethyl | -C≡C- | H | H | $CH_3$ | 4-F-phenyl | E | 1,15 (t), 1,99 (s), 4,68 (d), 6,08 (t) |
| 1.45 | 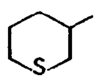 | Propyl | -C≡C- | H | H | $CH_3$ | 4-F-phenyl | E | 0,97 (t), 1,98 (s), 4,67 (d), 6,08 (t) |
| 1.46 | 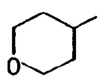 | Ethyl | -C≡C- | H | H | $CH_3$ | 4-F-phenyl | E | 1,14 (t), 1,97 (s), 3,37 (t), 3,99 (dd), 4,67 (d), 6,09 (t) |
| 1.47 | 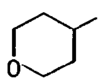 | Propyl | -C≡C- | H | H | $CH_3$ | 4-F-phenyl | E | |
| 1.48 | 1-$CH_3$S-cyclopropyl | Ethyl | -C≡C- | H | H | $CH_3$ | H | E | 0,74 (m), 1,13 (t), 1,88 (s), 2,09 (s), 2,9 (s), 4,63 (d) |
| 1.49 | 1-$CH_3$S-cyclopropyl | Propyl | -C≡C- | H | H | $CH_3$ | H | E | 0,74 (m), 0,98 (t), 1,9 (s), 2,12 (s), 2,9 (s), 4,63 (d) |

Tabelle 1 (Fortsetzung)

| Nr. | $R^1$ | $R^2$ | W | $R^3$ | $R^4$ | $R^5$ | $R^6$ | Konfig. | physik. Daten (300 MHz-$^1$H-NMR [ppm] *)) |
|---|---|---|---|---|---|---|---|---|---|
| 1.50 | 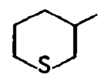 | Ethyl | -C≡C- | H | H | $CH_3$ | 1-OH-cyclohexyl | Z | 1,15 (t), 1,9 (s), 4,7 (d), 5,8 (m) |
| 1.51 | 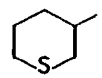 | Ethyl | -C≡C- | H | H | $CH_3$ | 4-Cl-phenyl | E | |
| 1.52 | 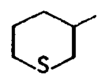 | Propyl | -C≡C- | H | H | $CH_3$ | 4-Cl-phenyl | E | |
| 1.53 | 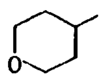 | Ethyl | -C≡C- | H | H | $CH_3$ | 4-Cl-phenyl | E | |
| 1.54 | 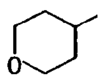 | Propyl | -C≡C- | H | H | $CH_3$ | 4-Cl-phenyl | E | |

Tabelle 2

$$H_2N-O-CH_2-CH=\underset{\displaystyle R^5}{\underset{|}{C}}-W-R^6 \qquad (III) \qquad (R^3,\ R^4 = H;\ W = -C\equiv C-\ \text{oder}\ -CH=CH-)$$

| Nr. | $R^5$ | $R^6$ | W | Konfig. | Ausbeute | physik. Daten (300 MHz-$^1$H-NMR [δ in ppm]/Fp.) |
|---|---|---|---|---|---|---|
| 2.01 | $CH_3$ | H | $-C\equiv C-$ | E | 90 % | Öl; 1,8 (s, 3H), 2,88 (s, 1H), 4,26 (d, 2H), 5,45 (s, 2H), 6,08 (t, 1H) |
| 2.02 | $CH_3$ | 4-F-phenyl | $-C\equiv C-$ | E | | Öl; 1,87 (s, 3H), 4,21 (d, 2H), 6,1 (m, 3H), 7,15-7,6 (2m, 4H) |
| 2.03 | $CH_3$ | 4-Cl-phenyl | $-C\equiv C-$ | E | | |
| 2.04 | $CH_3$ | H | $-C\equiv C-$ | Z | 63 % | 1,9 (s); 3,2 (s); 4,35 (d) |
| 2.05 | $CH_3$ | 4-F-phenyl | $-C\equiv C-$ | Z | | Öl; 1,99 (s, 3H), 4,23 (d, 2H), 5,45 (s, 2H), 5,91 (t, 1H), 6,9-7,5 (2m, 4H) |
| 2.06 | $CH_3$ | 4-Cl-phenyl | $-C\equiv C-$ | Z | 92 % | Öl; 2,0 (s, 3H), 4,45 (d, 2H), 5,4 (s, 2H), 5,91 (t, 1H), 7,2-7,5 (m, 4H) |
| 2.07 | H | 4-F-phenyl | E-CH=CH- | E | 91 % | 54-56°C, NMR: 4,26 (d, 2H), 5,4 (s, 2H), 5,8-6,0 (m, 1H), 6,3-6,8 (m, 3H), 6,9-7,5 (m, 4H) |
| 2.08 | H | $CH_3$ | E-CH=CH- | E | | |
| 2.09 | H | $C_2H_5$ | E-CH=CH- | E | | |
| 2.10 | $CH_3$ | 1-OH-cyclohexyl | $-C\equiv C-$ | Z | | 1,9 (s), 4,35 (d), 5,45 (bs), 5,8 (m) |
| 2.11 | $CH_3$ | 1-OH-2,2,6,6-$(CH_3)_4$-cyclohexyl | $-C\equiv C-$ | Z | | |

Tabelle 3

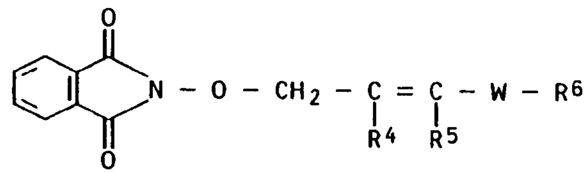

(IX)

($R^3$ = H; W = -C≡C- oder -CH=CH-)

| Nr. | $R^4$ | $R^5$ | $R^6$ | W | Konfig. | Ausbeute | physik. Daten (250 MHz-$^1$H-NMR [δ in ppm]/Fp.) |
|---|---|---|---|---|---|---|---|
| 3.01 | H | $CH_3$ | H | -C≡C- | Z | 48 % | 102-103°C; NMR: 1,85 (s,3H), 4,24 (s,1H), 4,82 (d,2H), 6,08 (t,1H), 7,88 (s,4H) |
| 3.02 | H | H | 4-F-phenyl | E-CH=CH- | E | 57 % | 155-157°C; NMR: 4,77 (d,2H), 5,9-6,1 (m,1H), 6,4-6,7 (m,2H), 6,8-7,0 (m,1H), 7,1-7,6 (2m,4H), 7,85 (s,4H) |
| 3.03 | H | $CH_3$ | 4-Cl-phenyl | -C≡C- | Z | 50 % | 109-111°C; NMR: 1,83 (s,3H), 4,92 (d,2H), 6,1 (t,1H), 7,3-7,55 (m,4H), 7,8 (s,4H) |
| 3.04 | H | $CH_3$ | H | -C≡C- | E | 66 % | 122-124°C; NMR: 1,84 (s,3H), 4,04 (s,1H), 4,77 (d,2H), 6,08 (t,1H), 7,8 (s,4H) |
| 3.05 | H | $CH_3$ | 1-OH-cyclohexyl | -C≡C- | Z | | NMR: 1,9 (s), 4,9 (d), 5,95 (m), 7,85 (m), |
| 3.06 | H | H | $CH_3$ | E-CH=CH- | E | | |
| 3.07 | H | H | $C_2H_5$ | E-CH=CH- | E | | |
| 3.08 | H | $CH_3$ | 1-OH-2,2,6,6-$(CH_3)_4$-Cyclohexyl | -C≡C- | Z | | |

Tabelle 3 (Fortsetzung)

| Nr. | $R^4$ | $R^5$ | $R^6$ | W | Konfig. | Ausbeute | physik. Daten (250 MHz-$^1$H-NMR [δ in ppm]/Fp.) |
|---|---|---|---|---|---|---|---|
| 3.09 | H | $CH_3$ | 4-F-phenyl | -C≡C- | Z | | 102-104°C; NMR: 1,93 (s,3H), 4,4 (d,2H), 6,09 (t,1H), 7,2-7,5 (2m,4H), 7,81 (s,4H) |
| 3.10 | H | $CH_3$ | 4-Cl-phenyl | -C≡C- | E | | |
| 3.11 | H | $CH_3$ | 4-F-phenyl | -C≡C- | E | | NMR: 1,96 (s,3H), 4,86 (d,2H), 6,16 (t,1H), 7,2-7,7 (2m,4H), 7,81 (s,4H) |

Beispiel 3

cis-5-(4-Chlorphenyl)-3-methyl-pent-2-en-4-in-1-ol

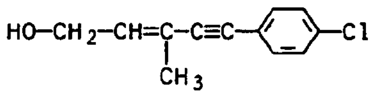

(Zwischenprodukt 4.02)

Zu einer Mischung aus 76,6 g (0,4 mol) 1-Chlor-4-Brombenzol, 1,2 g (1,7 mmol) $Pd[P(C_6H_5)_3]_2Cl_2$, 2,6 g (13 mmol) Kupfer(I)-jodid, 6 g (20 mmol) Triphenylphosphin und 200 ml Triethylamin wurden bei 50 bis 60°C innerhalb von 25 Minuten 46,1 g (0,48 mol) cis-3-Methyl-2-penten-4-in-1-ol getropft.

Nach 5 Stunden Erhitzen auf 90°C versetzte man das Reaktionsgemisch mit Methyl-tert.-butylether/Wasser und trennte anschließend die Phasen. Die organische Phase wurde mit gesättigter wäßriger Ammoniumchlorid-Lösung und dann mit gesättigter wäßriger Natriumhydrogencarbonat-Lösung gewaschen und wie üblich auf das Produkt hin aufgearbeitet. Ausbeute: 99,7 g (enthaltend 70 % Produkt).

Das Rohprodukt kann ohne weitere Reinigung zur Synthese der Cyclohexandione I verwendet werden.

Weitere wichtige Zwischenprodukte sind in Tabelle 4 aufgeführt.

Tabelle 4

$HO-CH_2-CH=C(CH_3)-W-R^6$ ($R^3$, $R^4$ = H; $R^5$ = $CH_3$; W = $-C\equiv C-$, $-CH=CH-$)

| Nr. | $R^6$ | W | Konfig. | physik.Daten |
|---|---|---|---|---|
| 4.01 | 4-F-phenyl | $-C\equiv C-$ | Z | |
| 4.02 | 4-Cl-phenyl | $-C\equiv C-$ | Z | |

Anwendungsbeispiele (herbizide Wirksamkeit)

Die herbizide Wirkung der ungesättigten Cyclohexenonoximether der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen bereits in den Versuchsgefäßen angezogen oder einige Tage vorher in die Versuchsgefäße verpflanzt. Die Applikation der in Wasser suspendierten oder emulgierten Wirkstoffe erfolgte je nach Wuchsform erst bei einer Wuchshöhe von 3 bis 15 cm. Die Aufwandmenge für die Nachauflaufbehandlung betrug 0,5 kg/ha a.S.

Die Pflanzen wurden artenspezifisch bei Temperaturen von 10-25°C bzw. 20-35°C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Botanischer Name | Deutscher Name |
|---|---|
| Avena sativa | Hafer (Ausfallkultur) |
| Echinochloa crus-galli | Hühnerhirse |

Das Ergebnis zeigte, daß sich mit den Verbindungen Nr. 1.01, 1.02 und 1.03 grasartige Pflanzen im Nachauflaufverfahren sehr gut bekämpfen lassen.

## Patentansprüche

1. Ungesättigte Cyclohexenonoximether der allgemeinen Formel I

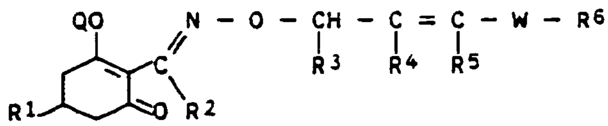

wobei die Variablen die folgende Bedeutung haben:

Q

Wasserstoff, eine $C_1$-$C_6$-Alkylcarbonylgruppe, die Benzoylgruppe, ein Alkalimetall- oder Erdalkalimetallion, ein Ammoniumion, dessen Stickstoffatom ein bis vier $C_1$-$C_4$-Alkyl-, Phenyl- und/oder Benzylsubstituenten tragen kann, ein Phosphonium-, Sulfonium- oder Sulfoxoniumion oder ein Äquivalent eines Übergangsmetallkations;

W

eine Gruppe -C≡C- oder -CH=CH-;

$R^1$

eine $C_1$-$C_4$-Alkoxy-$C_1$-$C_6$-alkyl- oder $C_1$-$C_4$-Alkylthio-$C_1$-$C_6$-alkylgruppe, eine $C_3$-$C_7$-Cycloalkyl- oder $C_5$-$C_7$-Cycloalkenylgruppe, eine 6-gliedrige heterocyclische Gruppe mit einem oder zwei nicht benachbarten Sauerstoff- und/oder Schwefelatomen, die gesättigt oder partiell ungesättigt sein kann, wobei die cyclischen Gruppen noch einen bis drei der folgenden Reste tragen können: Hydroxyl, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy oder $C_1$-$C_4$-Alkylthio;

ein 5-gliedriger gesättigter Heterocyclus mit einem oder zwei Sauerstoff- und/oder Schwefelatomen als Heteroatome, der noch einen bis drei der folgenden Reste tragen kann: $C_1$-$C_4$-Alkyl, partiell oder vollständig halogeniertes $C_1$-$C_4$-Alkyl, $C_1$-$C_4$-Alkoxy oder $C_1$-$C_4$-Alkylthio;

eine 5-gliedrige heteroaromatische Gruppe mit einem oder zwei Stickstoffatomen und/oder einem Sauerstoff- oder Schwefelatom, die noch einen bis drei der folgenden Reste tragen kann: Cyano, Halogen, $C_1$-$C_4$-Alkyl, partiell oder vollständig halogeniertes $C_1$-$C_4$ Alkyl, $C_2$-$C_6$-Alkenyl, partiell oder vollständig halogeniertes $C_2$-$C_6$-Alkenyl, $C_1$-$C_4$-Alkoxy, $C_1$-$C_4$-Alkylthio, $C_2$-$C_6$-Alkenyloxy oder $C_1$-$C_4$-Alkoxy-$C_1$-$C_4$-alkyl;

die Phenyl- oder Pyridylgruppe oder eine durch einen Rest $R^7$-x- substituierte $C_1$-$C_6$-Alkylgruppe, wobei X Sauerstoff, Schwefel, -SO- oder -$SO_2$- und $R^7$ $C_1$-$C_4$-Alkyl, Phenyl oder 5- oder 6-gliedriges Heteroaryl mit einem bis drei Heteroatomen, ausgewählt aus einer Gruppe von einem Sauerstoff- oder Schwefelatom und drei Stickstoffatomen, ausgenommen Verbindungen mit drei gleichzeitig benachbarten Heteroatomen im Heterocyclus, bedeutet und wobei der Aromat oder Heteroaromat dieser Gruppen noch einen bis drei der folgenden Substituenten tragen kann: Nitro, Cyano, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, $C_1$-$C_4$-Alkoxy-$C_1$-$C_4$-alkyl, $C_1$-$C_4$-Alkylthio-$C_1$-$C_4$-alkyl, $C_3$-$C_6$-Alkenyloxy, $C_3$-$C_6$-Alkinyloxy oder -$NR^8R^9$, wobei $R^8$ Wasserstoff, $C_1$-$C_4$-Alkyl, $C_3$-$C_4$-Alkenyl oder $C_3$-$C_4$-Alkinyl und $R^9$ Wasserstoff, $C_1$-$C_4$-Alkyl, $C_3$-$C_4$-Alkenyl, $C_3$-$C_4$-Alkinyl, $C_1$-$C_6$-Alkylcarbonyl oder Benzoyl, das zusätzlich noch einen bis drei der folgenden Reste tragen kann: Nitro, Cyano, Halogen, $C_1$-$C_4$-Alkyl, partiell oder vollständig halogeniertes $C_1$-$C_4$-Alkyl, $C_1$-$C_4$-Alkoxy oder $C_1$-$C_4$-Alkylthio, bedeuten;

$R^2$

eine $C_1$-$C_6$-Alkylgruppe;

$R^3$
Wasserstoff oder eine $C_1$-$C_6$-Alkylgruppe;
$R^4$
Wasserstoff, Halogen oder eine $C_1$-$C_4$-Alkylgruppe;
$R^5$
Wasserstoff oder eine $C_1$-$C_6$-Alkylgruppe;
oder $R^3$ und $R^4$, $R^3$ und $R^5$ oder $R^4$ und $R^5$ bilden zusammen eine $C_2$-$C_4$-Alkylen- oder $C_2$-$C_4$-Alkenylenkette;
$R^6$
Wasserstoff, eine $C_1$-$C_6$-Alkylgruppe, eine partiell oder vollständig halogenierte $C_1$-$C_4$-Alkylgruppe, die noch einen Phenylrest tragen kann, eine $C_2$-$C_6$-Alkenylgruppe, eine $C_1$-$C_4$-Alkoxy-$C_1$-$C_4$-alkylgruppe, eine $C_1$-$C_4$-Alkylthio-$C_1$-$C_4$-alkylgruppe, eine $C_3$-$C_7$-Cycloalkyl- oder $C_5$-$C_7$-Cycloalkenylgruppe, die beide noch einen bis drei der folgenden Reste tragen können: Hydroxyl, $C_1$-$C_4$-Alkyl, partiell oder vollständig halogeniertes $C_1$-$C_4$-Alkyl, $C_1$-$C_4$-Alkoxy, $C_1$-$C_4$-Alkoxy-$C_1$-$C_4$-alkyl oder $C_1$-$C_4$-Alkylthio-$C_1$-$C_4$-alkyl;
die Phenyl- oder die Pydridylgruppe, die beide noch einen bis drei der folgenden Reste tragen können: Nitro, Cyano, Hydroxyl, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy ; $C_1$-$C_4$-Alkylthio, $C_1$-$C_4$-Alkoxy-$C_1$-$C_4$-alkyl oder $C_1$-$C_4$-Alkylthio-$C_1$-$C_4$-alkyl.

2. Herbizides Mittel, enthaltend einen flüssigen oder festen Trägerstoff und mindestens einen ungesättigten Cyclohexenonoximether I gemäß Anspruch 1.

3. Verfahren zur Bekämpfung von unerwünschtem Pflanzenwuchs, dadurch gekennzeichnet, daß man eine herbizid wirksame Menge eines ungesättigten Cyclohexenonoximethers I gemäß Anspruch 1 auf Pflanzen, deren Lebensraum oder auf Saatgut einwirken läßt.

4. Hydroxylamine der allgemeinen Formel IIIb

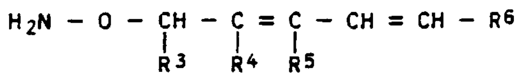

IIIb

wobei die Substituenten die folgende Bedeutung haben:
$R^3$
Wasserstoff oder eine $C_1$-$C_6$-Alkylgruppe;
$R^4$
Wasserstoff, Halogen oder eine $C_1$-$C_6$-Alkylgruppe;
$R^5$
Wasserstoff oder eine $C_1$-$C_6$-Alkylgruppe;
oder $R^3$ und $R^4$, $R^3$ und $R^5$ oder $R^4$ und $R^5$ bilden zusammen eine $C_2$-$C_4$-Alkylen- oder $C_2$-$C_4$-Alkenylenkette;
$R^6$
Wasserstoff, eine $C_1$-$C_6$-Alkylgruppe, eine partiell oder vollständig halogenierte $C_1$-$C_4$-Alkylgruppe, die noch einen Phenylrest tragen kann, eine $C_2$-$C_6$-Alkenylgruppe, eine $C_1$-$C_4$-Alkoxy-$C_1$-$C_4$-alkylgruppe, eine $C_1$-$C_4$-Alkylthio-$C_1$-$C_4$-alkylgruppe, eine $C_3$-$C_7$-Cycloalkyl- oder $C_5$-$C_7$-Cycloalkenylgruppe, die beide noch einen bis drei der folgenden Reste tragen können: Hydroxyl, $C_1$-$C_4$-Alkyl, partiell oder vollständig halogeniertes $C_1$-$C_4$-Alkyl, $C_1$-$C_4$-Alkoxy, $C_1$-$C_4$-Alkoxy-$C_1$-$C_4$-alkyl oder $C_1$-$C_4$-Alkylthio-$C_1$-$C_4$-alkyl;
die Phenyl- oder die Pydridylgruppe, die beide noch einen bis drei der folgenden Reste tragen können: Nitro, Cyano, Hydroxyl, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, $C_1$-$C_4$-Alkoxy-$C_1$-$C_4$-alkyl oder $C_1$-$C_4$-Alkylthio-$C_1$-$C_4$-alkyl.

5. Phthalimide der allgemeinen Formel IX

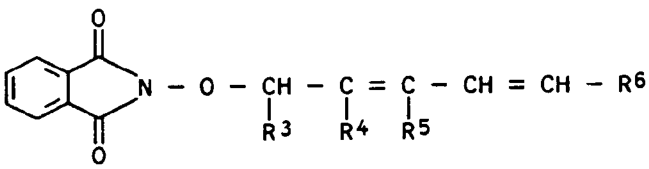

IX

wobei die Variablen die folgende Bedeutung haben:

$R^3$

Wasserstoff oder eine $C_1$-$C_6$-Alkylgruppe;

$R^4$

Wasserstoff, Halogen oder eine $C_1$-$C_6$-Alkylgruppe;

$R^5$

Wasserstoff oder eine $C_1$-$C_6$-Alkylgruppe;

oder $R^3$ und $R^4$, $R^3$ und $R^5$ oder $R^4$ und $R^5$ bilden zusammen eine $C_2$-$C_4$-Alkylen- oder $C_2$-$C_4$-Alkenylenkette;

$R^6$

Wasserstoff, eine $C_1$-$C_6$-Alkylgruppe, eine partiell oder vollständig halogenierte $C_1$-$C_4$-Alkylgruppe, die noch einen Phenylrest tragen kann, eine $C_2$-$C_6$-Alkenylgruppe, eine $C_1$-$C_4$-Alkoxy-$C_1$-$C_4$-alkylgruppe, eine $C_1$-$C_4$-Alkylthio-$C_1$-$C_4$-alkylgruppe, eine $C_3$-$C_7$-Cycloalkyl- oder $C_5$-$C_7$-Cycloalkenylgruppe, die beide noch einen bis drei der folgenden Reste tragen können: Hydroxyl, $C_1$-$C_4$-Alkyl, partiell oder vollständig halogeniertes $C_1$-$C_4$-Alkyl, $C_1$-$C_4$-Alkoxy, $C_1$-$C_4$-Alkoxy-$C_1$-$C_4$-alkyl oder $C_1$-$C_4$-Alkylthio-$C_1$-$C_4$-alkyl;

die Phenyl- oder die Pydridylgruppe, die beide noch einen bis drei der folgenden Reste tragen können: Nitro, Cyano, Hydroxyl, Halogen, $C_1$-$C_4$-Alkyl, partiell oder vollständig halogeniertes $C_1$-$C_4$-Alkyl, $C_1$-$C_4$-Alkoxy, partiell oder vollständig halogeniertes $C_1$-$C_4$-Alkoxy, $C_1$-$C_4$-Alkylthio, $C_1$-$C_4$-Alkoxy-$C_1$-$C_4$-alkyl oder $C_1$-$C_4$-Alkylthio-$C_1$-$C_4$-alkyl.

6. Phthalimide der Formel IX nach Anspruch 5, wobei $R^3$ bis $R^5$ Wasserstoff bedeuten.

## Claims

1. An unsaturated cyclohexenone oxime ether of the formula I

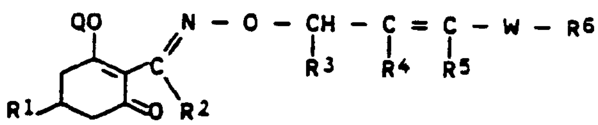

where

Q

is hydrogen, $C_1$-$C_6$-alkylcarbonyl, benzoyl, an alkali metal or alkaline earth metal ion, an ammonium ion whose nitrogen can carry from one to four $C_1$-$C_4$-alkyl, phenyl and/or benzyl substituents, or a phosphonium, sulfonium or sulfoxonium ion or an equivalent of a transition metal cation;

W

is -C≡C- or -CH=CH-;

$R^1$

is $C_1$-$C_4$-alkoxy-$C_1$-$C_6$-alkyl or $C_1$-$C_4$-alkylthio-$C_1$-$C_6$-alkyl, $C_3$-$C_7$-cycloalkyl or $C_5$-$C_7$-cycloalkenyl, a 6-membered heterocyclic group which has one or two non-adjacent oxygen and/or sulfur atoms and can be saturated or partially unsaturated, it also being possible for the cyclic groups to carry one to three of the following: hydroxyl, halogen, $C_1$-$C_4$-alkyl, partially or completely halogenated $C_1$-$C_4$-alkyl, $C_1$-$C_4$-alkoxy, partially or completely halogenated $C_1$-$C_4$-alkoxy or $C_1$-$C_4$-alkylthio; a 5-membered saturated heterocycle which has one or two oxygen and/or sulfur atoms as hetero atoms and can also carry one to three of the following: $C_1$-$C_4$-alkyl, partially or completely halogenated $C_1$-$C_4$-alkyl, $C_1$-$C_4$-alkoxy or $C_1$-$C_4$-alkylthio; a 5-membered heteroaromatic group which has one or two nitrogen atoms and/or one oxygen or sulfur atom and can also carry one to three of the following: cyano, halogen, $C_1$-$C_4$-alkyl, partially or completely halogenated $C_1$-$C_4$-alkyl, $C_2$-$C_6$-alkenyl, partially or completely halogenated $C_2$-$C_6$-alkenyl, $C_1$-

$C_4$-alkoxy, $C_1$-$C_4$-alkylthio, $C_2$-$C_6$-alkenyloxy or $C_1$-$C_4$-alkoxy-$C_1$-$C_4$-alkyl; phenyl or pyridyl, or $C_1$-$C_6$-alkyl which is substituted by $R^7$-X- where X is oxygen, sulfur, -SO- or -$SO_2$- and $R^7$ is $C_1$-$C_4$-alkyl, phenyl or 5- or 6-membered hetaryl with one to three hetero atoms selected from the group comprising one oxygen or sulfur atom and three nitrogen atoms, excepting compounds with three adjacent hetero atoms in the heterocycle, and where the aromatic or heteroaromatic moiety of these groups can also carry one to three of the following: nitro, cyano, halogen, $C_1$-$C_4$-alkyl, partially or completely halogenated $C_1$-$C_4$-alkyl, $C_1$-$C_4$-alkoxy, partially or completely halogenated $C_1$-$C_4$-alkoxy, $C_1$-$C_4$-alkylthio, $C_1$-$C_4$-alkoxy-$C_1$-$C_4$-alkyl, $C_1$-$C_4$-alkylthio-$C_1$-$C_4$-alkyl, $C_3$-$C_6$-alkenyloxy, $C_3$-$C_6$-alkynyloxy or $NR^8R^9$ where $R^8$ is hydrogen, $C_1$-$C_4$-alkyl, $C_3$-$C_4$-alkenyl or $C_3$-$C_4$-alkynyl and $R^9$ is hydrogen, $C_1$-$C_4$-alkyl, $C_3$-$C_4$-alkenyl, $C_3$-$C_4$-alkynyl, $C_1$-$C_6$-alkylcarbonyl or benzoyl which can also carry one to three of the following: nitro, cyano, halogen, $C_1$-$C_4$-alkyl, partially or completely halogenated $C_1$-$C_4$-alkyl, $C_1$-$C_4$-alkoxy or $C_1$-$C_4$-alkylthio;
$R^2$
is $C_1$-$C_6$-alkyl;
$R^3$
is hydrogen or $C_1$-$C_6$-alkyl;
$R^4$
is hydrogen, halogen or $C_1$-$C_4$-alkyl;
$R^5$
is hydrogen or $C_1$-$C_6$-alkyl;
or $R^3$ and $R^4$, $R^3$ and $R^5$ or $R^4$ and $R^5$ together form $C_2$-$C_4$-alkylene or $C_2$-$C_4$-alkenylene;
$R^6$
is hydrogen, $C_1$-$C_6$-alkyl, partially or completely halogenated $C_1$-$C_4$-alkyl which can also carry a phenyl radical, or $C_2$-$C_6$-alkenyl, $C_1$-$C_4$-alkoxy-$C_1$-$C_4$-alkyl, $C_1$-$C_4$-alkylthio-$C_1$-$C_4$-alkyl, $C_3$-$C_7$-cycloalkyl or $C_5$-$C_7$-cycloalkenyl, both of which can also carry one to three of the following: hydroxyl, $C_1$-$C_4$-alkyl, partially or completely halogenated $C_1$-$C_4$-alkyl, $C_1$-$C_4$-alkoxy, $C_1$-$C_4$-alkoxy-$C_1$-$C_4$-alkyl or $C_1$-$C_4$-alkylthio-$C_1$-$C_4$-alkyl;
phenyl or pyridyl, both of which can also carry one to three of the following: nitro, cyano, hydroxyl, halogen, $C_1$-$C_4$-alkyl, partially or completely halogenated $C_1$-$C_4$-alkyl, $C_1$-$C_4$-alkoxy, partially or completely halogenated $C_1$-$C_4$-alkoxy, $C_1$-$C_4$-alkylthio, $C_1$-$C_4$-alkoxy-$C_1$-$C_4$-alkyl or $C_1$-$C_4$-alkylthio-$C_1$-$C_4$-alkyl.

2. A herbicidal agent containing a liquid or solid carrier and at least one unsaturated cyclohexenone oxime ether I as claimed in claim 1.

3. A method for controlling unwanted plant growth, which comprises allowing a herbicidally effective amount of an unsaturated cyclohexenone oxime ether I as claimed in claim 1 to act on plants, their habitat or on seeds.

4. A hydroxylamine of the formula IIIb

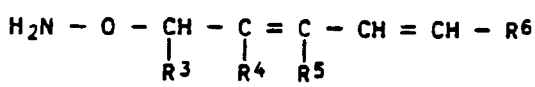

IIIb

where
$R^3$
is hydrogen or $C_1$-$C_6$-alkyl;
$R^4$
is hydrogen, halogen or $C_1$-$C_6$-alkyl;
$R^5$
is hydrogen or C1-$C_6$-alkyl;
or $R^3$ and $R^4$, $R^3$ and $R^5$ or $R^4$ and $R^5$ together form $C_2$-$C_4$-alkylene or $C_2$-$C_4$-alkenylene;
$R^6$
is hydrogen, C1-$C_6$-alkyl, partially or completely halogenated $C_1$-$C_4$-alkyl which can also carry a phenyl radical, or $C_2$-$C_6$-alkenyl, $C_1$-$C_4$-alkoxy-$C_1$-$C_4$-alkyl, $C_1$-$C_4$-alkylthio-$C_1$-$C_4$-alkyl, $C_3$-$C_7$-cycloalkyl or $C_5$-$C_7$-cycloalkenyl, both of which can also carry one to three of the following: hydroxyl, $C_1$-$C_4$-alkyl, partially or completely halogenated $C_1$-$C_4$-alkyl, $C_1$-$C_4$-alkoxy, $C_1$-$C_4$-alkoxy-$C_1$-$C_4$-alkyl or $C_1$-$C_4$-alkylthio-$C_1$-$C_4$-alkyl;

phenyl or pyridyl, both of which can also carry one to three of the following: nitro, cyano, hydroxyl, halogen, $C_1$-$C_4$-alkyl, partially or completely halogenated $C_1$-$C_4$-alkyl, $C_1$-$C_4$-alkoxy, partially or completely halogenated $C_1$-$C_4$-alkoxy, $C_1$-$C_4$-alkylthio, $C_1$-$C_4$-alkoxy-$C_1$-$C_4$-alkyl or $C_1$-$C_4$-alkylthio-$C_1$-$C_4$-alkyl.

5. A phthalimide of the formula IX

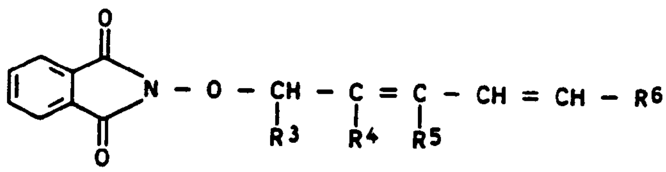

IX

where
$R^3$
is hydrogen or $C_1$-$C_6$-alkyl;
$R^4$
is hydrogen, halogen or $C_1$-$C_6$-alkyl;
$R^5$
is hydrogen or $C_1$-$C_6$-alkyl;
or $R^3$ and $R^4$, $R^3$ and $R^5$ or $R^4$ and $R^5$ together form $C_2$-$C_4$-alkylene or $C_2$-$C_4$-alkenylene;
$R^6$
is hydrogen, $C_1$-$C_6$-alkyl, partially or completely halogenated $C_1$-$C_4$-alkyl which can also carry a phenyl radical, or $C_2$-$C_6$-alkenyl, $C_1$-$C_4$-alkoxy-$C_1$-$C_4$-alkyl , $C_1$-$C_4$-alkylthio-$C_1$-$C_4$-alkyl, $C_3$-$C_7$-cycloalkyl or $C_5$-$C_7$-cycloalkenyl, both of which can also carry one to three of the following: hydroxyl, $C_1$-$C_4$-alkyl, partially or completely halogenated $C_1$-$C_4$-alkyl, $C_1$-$C_4$-alkoxy, $C_1$-$C_4$-alkoxy-$C_1$-$C_4$-alkyl or $C_1$-$C_4$-alkylthio-$C_1$-$C_4$-alkyl;
phenyl or pyridyl, both of which can also carry one to three of the following: nitro, cyano, hydroxyl, halogen, $C_1$-$C_4$-alkyl, partially or completely halogenated $C_1$-$C_4$-alkyl, $C_1$-$C_4$-alkoxy, partially or completely halogenated $C_1$-$C_4$-alkoxy, $C_1$-$C_4$-alkylthio, $C_1$-$C_4$-alkoxy-$C_1$-$C_4$-alkyl or $C_1$-$C_4$-alkylthio-$C_1$-$C_4$-alkyl.

6. A phthalimide of the formula IX as claimed in claim 5, where $R^3$ to $R^5$ are hydrogen.

## Revendications

1. Ethers insaturés de cyclohexénonoximes de formule générale I

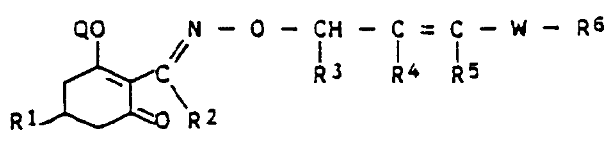

dans laquelle le symboles ont les significations suivantes :
Q représente l'hydrogène, un groupe (alkyle en $C_1$-$C_6$)-carbonyle, le groupe benzoyle, un ion de métal alcalin ou alcalino-terreux, un ion ammonium dont l'atome d'azote peut porter 1 à 4 substituants alkyle en $C_1$-$C_4$, phényle et/ou benzyle, un ion phosphonium, sulfonium ou sulfoxonium ou un équivalent d'un cation de métal de transition ;
W représente un groupe -C≡C- ou -CH=CH- ;
$R^1$ représente un groupe (alcoxy en $C_1$-$C_4$)-alkyle en $C_1$-$C_6$ ou (alkylthio en $C_1$-$C_4$)-alkyle en $C_1$-$C_6$, un groupe cycloalkyle en $C_3$-$C_7$ ou cycloalcényle en $C_5$-$C_7$, un groupe hétérocyclique à 6 chaînons contenant un ou deux atomes d'oxygène et/ou de soufre non voisins, qui peut être saturé ou partiellement insaturé, les groupes cycliques pouvant encore porter 1 à 3 des substituants suivants : hydroxy, halogéno, alkyle en $C_1$-$C_4$, alkyle en $C_1$-$C_4$ halogéné en totalité ou en partie, alcoxy en $C_1$-$C_4$, alcoxy en $C_1$-$C_4$ halogéné en totalité ou en partie ou alkylthio en $C_1$-$C_4$ ;
un hétérocycle saturé à 5 chaînons contenant un ou deux atomes d'oxygène et/ou de soufre en tant qu'hétéroatomes, et qui peut encore porter 1 à 3 des substituants suivants : alkyle en $C_1$-$C_4$, alkyle en $C_1$-$C_4$

halogéné en totalité ou en partie, alcoxy en $C_1$-$C_4$ ou alkylthio en $C_1$-$C_4$ ;
un groupe hétéroaromatique à 5 chaînons contenant un ou deux atomes d'azote et/ou un atome d'oxygène ou de soufre, et qui peut encore porter 1 à 3 des substituants suivants : cyano, halogéno, alkyle en $C_1$-$C_4$, alkyle en $C_1$-$C_4$ halogéné en totalité ou en partie, alcényle en $C_2$-$C_6$, alcényle en $C_2$-$C_6$ halogéné en totalité ou en partie, alcoxy en $C_1$-$C_4$, alkylthio en $C_1$-$C_4$, alcényloxy en $C_2$-$C_6$ ou (alcoxy en $C_1$-$C_4$)-alkyle en $C_1$-$C_4$ ; un groupe phényle ou pyridyle ou un groupe alkyle en $C_1$-$C_6$ substitué par un groupe $R^7$-X- dans lequel X représente l'oxygène, le soufre, -SO- ou -$SO_2$- et $R^7$ représente un groupe alkyle en $C_1$-$C_4$, phényle ou un groupe hétéroaryle à 5 ou 6 chaînons contenant 1 à 3 hétéroatomes choisis dans un groupe d'un atome d'oxygène ou de soufre et 3 atomes d'azote, à l'exception des composés contenant trois hétéroatomes voisins dans l'hétérocycle, la partie aromatique ou hétéroaromatique de ces groupes pouvant encore porter 1 à 3 atomes des substituants suivants : nitro, cyano, halogéno, alkyle en $C_1$-$C_4$, alkyle en $C_1$-$C_4$ halogéné en totalité ou en partie ; alcoxy en $C_1$-$C_4$ halogéné en totalité ou en partie, alkylthio en $C_1$-$C_4$, (alcoxy en $C_1$-$C_4$)-alkyle en $C_1$-$C_4$, (alkylthio en $C_1$-$C_4$)-alkyle en $C_1$-$C_4$, alcényloxy en $C_3$-$C_6$, alcynyloxy en $C_3$-$C_6$ ou -$NR^8R^9$ dans lequel $R^8$ représente l'hydrogène, un groupe alkyle en $C_1$-$C_4$, alcényle en $C_3$-$C_4$ ou alcynyle en $C_3$-$C_4$ et $R^9$ représente l'hydrogène, un groupe alkyle en $C_1$-$C_4$, alcényle en $C_3$-$C_4$, alcynyle en $C_3$-$C_4$, (alkyle en $C_1$-$C_6$)-carbonyle ou benzoyle, lequel peut encore porter 1 à 3 des substituants suivants : nitro, cyano, halogéno, alkyle en $C_1$-$C_4$, alkyle en $C_1$-$C_4$ halogéné en totalité ou en partie, alcoxy en $C_1$-$C_4$ ou alkylthio en $C_1$-$C_4$ ;
$R^2$ représente un groupe alkyle en $C_1$-$C_6$ ;
$R^3$ représente l'hydrogène ou un groupe alkyle en $C_1$-$C_6$ ;
$R^4$ représente l'hydrogène, un halogène ou un groupe alkyle en $C_1$-$C_4$ ;
$R^5$ représente l'hydrogène ou un groupe alkyle en $C_1$-$C_6$ ;
ou bien $R^3$ et $R^4$, $R^3$ et $R^5$ ou $R^4$ et $R^5$ forment ensemble une chaîne alkylène en $C_2$-$C_4$ ou alcénylène en $C_2$-$C_4$ ;
$R^6$ représente l'hydrogène, un groupe alkyle en $C_1$-$C_6$, un groupe alkyle en $C_1$-$C_4$ halogéné en totalité ou en partie et qui peut encore porter un groupe phényle, un groupe alcényle en $C_2$-$C_6$, un groupe (alcoxy en $C_1$-$C_4$)-alkyle en $C_1$-$C_4$, un groupe (alkylthio en $C_1$-$C_4$)-alkyle en $C_1$-$C_4$, un groupe cycloalkyle en $C_3$-$C_7$ ou cycloalcényle en $C_5$-$C_7$ qui peut encore porter 1 à 3 des substituants suivants : hydroxy, alkyle en $C_1$-$C_4$, alkyle en $C_1$-$C_4$ halogéné en totalité ou en partie, alcoxy en $C_1$-$C_4$, (alcoxy en $C_1$-$C_4$)-alkyle en $C_1$-$C_4$ ou (alkylthio en $C_1$-$C_4$)-alkyle en $C_1$-$C_4$ ;
un groupe phényle ou pyridyle qui peut porter encore 1 à 3 des substituants suivants : nitro, cyano, hydroxy, halogéno, alkyle en $C_1$-$C_4$, alkyle en $C_1$-$C_4$ halogéné en totalité ou en partie, alcoxy en $C_1$-$C_4$, alcoxy en $C_1$-$C_4$ halogéné en totalité ou en partie, alkylthio en $C_1$-$C_4$, (alcoxy en $C_1$-$C_4$)-alkyle en $C_1$-$C_4$ ou (alkylthio en $C_1$-$C_4$)-alkyle en $C_1$-$C_4$.

2. Produit herbicide contenant un véhicule liquide ou solide et au moins un éther insaturé de cyclohexénonoxime I de la revendication 1.

3. Procédé pour combattre les croissances de végétaux indésirables caractérisé en ce que l'on fait agir sur les végétaux, leur habitat ou les semences une quantité herbicide efficace d'un éther insaturé de cyclohexénonoxime I de la revendication 1.

4. Hydroxylamines de formule générale IIIb

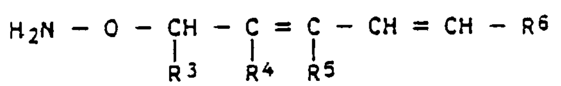

IIIb

dans laquelle les symboles ont les significations suivantes :
$R^3$ représente l'hydrogène ou un groupe alkyle en $C_1$-$C_6$ ;
$R^4$ représente l'hydrogène, un halogène ou un groupe alkyle en $C_1$-$C_6$ ;
$R^5$ représente l'hydrogène ou un groupe alkyle en $C_1$-$C_6$ ;
ou bien $R^3$ et $R^4$, $R^3$ et $R^5$ ou $R^4$ et $R^5$ forment ensemble un groupe alkylène en $C_2$-$C_4$ ou alcénylène en $C_2$-$C_4$ ;
$R^6$ représente l'hydrogène, un groupe alkyle en $C_1$-$C_6$, un groupe alkyle en $C_1$-$C_4$ halogéné en totalité ou en partie et qui peut encore porter un groupe phényle, un groupe alcényle en $C_2$-$C_6$, un groupe (alcoxy en $C_1$-$C_4$)-alkyle en $C_1$-$C_4$, un (alkylthio en $C_1$-$C_4$)-alkyle en $C_1$-$C_4$, un groupe cycloalkyle en $C_3$-$C_7$ ou cycloalcényle en $C_5$-$C_7$ qui peut porter encore 1 à 3 des substituants suivants : hydroxy, alkyle en $C_1$-$C_4$,

alkyle en $C_1$-$C_4$ halogéné en totalité ou en partie, alcoxy en $C_1$-$C_4$, (alcoxy en $C_1$-$C_4$)-alkyle en $C_1$-$C_4$ ou (alkylthio en $C_1$-$C_4$)-alkyle en $C_1$-$C_4$ ;
un groupe phényle ou pyridyle qui peut porter encore 1 à 3 des substituants suivants : nitro, cyano, hydroxy, halogéno, alkyle en $C_1$-$C_4$, alkyle en $C_1$-$C_4$ halogéné en totalité ou en partie, alcoxy en $C_1$-$C_4$, alcoxye en $C_1$-$C_4$ halogéné en totalité ou en partie, alkylthio en $C_1$-$C_4$, (alcoxy en $C_1$-$C_4$)-alkyle en $C_1$-$C_4$ ou (alkylthio en $C_1$-$C_4$)-alkyle en $C_1$-$C_4$.

**5.** Phtalimides de formules générale IX

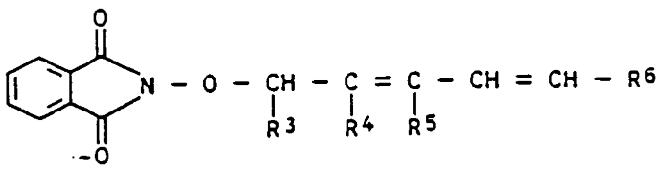

IX

dans laquelle les symboles ont les significations suivantes :
$R^3$ représente l'hydrogène ou un groupe alkyle en $C_1$-$C_6$ ;
$R^4$ représente l'hydrogène, un halogène ou un groupe alkyle en $C_1$-$C_6$ ;
$R^5$ représente l'hydrogène ou un groupe alkyle en $C_1$-$C_6$ ;
ou bien $R^3$ et $R^4$, $R^3$ et $R^5$ ou $R^4$ et $R^5$ forment ensemble un groupe alkylène en $C_2$-$C_4$ ou alcénylène en $C_2$-$C_4$ ;
$R^6$ représente l'hydrogène, un groupe alkyle en $C_1$-$C_6$, un groupe alkyle en $C_1$-$C_4$ halogéné en totalité ou en partie et qui peut encore porter un groupe phényle, un groupe alcényle en $C_2$-$C_6$, un groupe (alcoxy en $C_1$-$C_4$)-alkyle en $C_1$-$C_4$, un groupe (alkylthio en $C_1$-$C_4$)-alkyle en $C_1$-$C_4$, un groupe cycloalkyle en $C_3$-$C_7$ ou cycloalcényle en $C_5$-$C_7$ qui peut porter encore 1 à 3 des substituants suivants : hydroxy, alkyle en $C_1$-$C_4$, alkyle en $C_1$-$C_4$ halogéné en totalité ou en partie, alcoxy en $C_1$-$C_4$, (alcoxy en $C_1$-$C_4$)-alkyle en $C_1$-$C_4$ ou (alkylthio en $C_1$-$C_4$)-alkyle en $C_1$-$C_4$ ;
un groupe phényle ou pyridyle qui peut porter encore 1 à 3 des substituants suivants : nitro, cyano, hydroxy, halogéno, alkyle en $C_1$-$C_4$, alkyle en $C_1$-$C_4$ halogéné en totalité ou en partie, alcoxy en $C_1$-$C_4$, alcoxy en $C_1$-$C_4$ halogéné en totalité ou en partie, alkylthio en $C_1$-$C_4$, (alcoxy en $C_1$-$C_4$)-alkyle en $C_1$-$C_4$ ou (alkylthio en $C_1$-$C_4$)-alkyle en $C_1$-$C_4$.

**6.** Phtalimides de formule IX de la revendication 5, pour lesquels $R^3$ et $R^5$ représentent l'hydrogène.